# FASCICULE DE BREVET EUROPEEN

(11) **EP 2 734 681 B1**
(45) Date de publication et mention de la délivrance du brevet: **01.09.2021**
(21) Numéro de dépôt: 12751090.7
(22) Date de dépôt: 18.07.2012
(51) Int. Cl.: E04B 2/88

(54) **MUR RIDEAU INTEGRE SERVANT A LA PRODUCTION INDUSTRIELLE OPTIMISEE DE MICROALGUES EN FACADES DE BATIMENTS**
INTEGRIERTE VORHANGWAND ZUR OPTIMIERTEN INDUSTRIELLEN HERSTELLUNG VON MIKROALGEN AUF GEBÄUDEFASSADEN
INTEGRATED CURTAIN WALLING SERVING FOR OPTIMIZED INDUSTRIAL PRODUCTION OF MICROALGAE IN BUILDING FACADES

(30) Priorité: 18.07.2011 FR 1156521
(43) Date de publication de la demande: 28.05.2014
(73) Titulaire: X-TU, 75010 Paris (FR); Centre National de la Recherche Scientifique (C.N.R.S.), 75794 Paris Cedex 16 (FR); Université de Nantes, 44000 Nantes (FR)
(72) Inventeur: LEGENDRE, Anouk, F-75010 Paris (FR); DESMAZIÈRES, Nicolas, F-75010 Paris (FR); LEGRAND, Jack, F-44600 Saint-nazaire (FR); PRUVOST, Jérémy, F-44250 Saint Brevin Les Pins (FR)
(74) Mandataire: Novagraaf Technologies
(86) Numéro de dépôt international: PCT/FR2012/051704
(87) Numéro de publication internationale: WO 2013/011240

(56) Documents cités:
- EP-A1- 2 359 682
- WO-A2-2005/101525
- WO-A2-2010/072925
- JP-A- 2000 320 041
- None

## Description

La présente invention se rapporte à un module (D) photobioréacteur (PBR) d'isolation et/ou de régulation thermique ainsi qu'à l'utilisation de ce dispositif pour la fabrication d'un bâtiment. La présente invention se rapporte également à une façade de bâtiment comprenant un module (D) photobioréacteur (PBR) d'isolation et/ou de régulation thermique et à un bâtiment comprenant plusieurs modules (D) photobioréacteur (PBR) d'isolation et/ou de régulation thermique

La présente décrit également un dispositif d'isolation et/ou de régulation thermique pour le bâtiment ainsi que l'utilisation de ce dispositif pour la production industrielle de microalgues et/ou de microorganismes.

La présente invention trouve notamment des applications dans les domaines de l'architecture, de l'aménagement extérieur et intérieur, ou du paysagisme.

### Etat de la technique

Il existe actuellement un grand intérêt pour trouver des solutions alternatives aux énergies fossiles dans la mesure où ces énergies sont épuisables et sont à l'origine de nombreuses pollutions de l'environnement.

Par exemples, les villes polluent et produisent beaucoup de gaz carbonique par leurs centrales thermiques, par les chaufferies des bâtiments, dans les centrales de production de froid. Par ailleurs, le traitement de l'air vicié des bâtiments n'est pas toujours évident, notamment lorsqu'il est issu des humains, mais aussi des parkings souterrains, notamment des automobiles.

En particulier, du gaz carbonique (CO₂) et de l'oxyde d'azote (NO₂) s'échappent dans l'atmosphère et contribue à l'effet de serre et à la dégradation du climat.

Les villes consomment aussi beaucoup d'énergie, qui est produite à distance, et doit être transportée à grands frais avec une importante perte de charge même pour l'énergie électrique. Les quartiers d'affaires intégralement climatisés et fonctionnant à l'énergie électrique via des centrales thermiques sont un gouffre à énergie.

De nombreuses solutions ont été étudiées pour améliorer l'isolation des bâtiments, par exemple les systèmes d'isolation extérieure, la fabrication de vitre à isolation renforcée, par exemple des vitres double/triple vitrage.

En outre, des solutions ont été également étudié afin de diminuer la consommation d'énergies fossiles et/ou de trouver des solutions alternatives à ces énergies. Il s'agit par exemple de la production de moteurs hybrides électrique/thermique, de la production de moteurs électriques utilisant des batteries lithium, de la production de « biocarburant » à partir de microorganismes/plantes etc.

Le document WO2010/072925 décrit un dispositif comprenant un contenant de culture d'algues et/ou microorganismes, un moyen d'alimentation, un moyen d'injection d'un effluent, un moyen de régulation de la température, un moyen de connexion au bâtiment. Le contenant peut être un tube, panneau, notamment multicouche.

Le document JP 2000320041 décrit un dispositif comprenant un support comprenant une dépression dans la partie supérieure de celui-ci, un tuyau d'alimentation d'une couche de culture d'algues formant une couche de culture située dans la dépression et un tuyau de récupération de la culture comprenant une vanne permettant de définir la quantité de culture récupérée.

Le document EP 2 359 682 décrit des bioréacteurs plats pour attachement/intégration à un bâtiment. Ce document décrit également un bioréacteur cuboïde. Ce document décrit également l'intégration de bioréacteurs dans la façade de bâtiments formant ainsi le mur extérieur, par exemple via des rails, support etc. Enfin, ce document décrit en outre un bioréacteur plat constituant un élément de façade, ledit élément de façade étant inclinable, par exemple afin d'absorber le rayonnement solaire, l'élément de façade étant fixé via une structure de support.

Le document WO2005101525 décrit un dispositif comprenant l'association de panneaux solaires, comprenant des cellules voltaïques avec, notamment des photobioréacteurs. En particulier, ce document décrit un dispositif comprenant un photobioréacteur capable de transmettre la lumière et un élément comprenant des cellules photovoltaïques, ledit élément étant disposé entre la source lumineuse et le photobioréacteur. Ce document décrit également un bâtiment comprenant des éléments de façades qui sont des panneaux solaires qui laissent passer la lumière pour des photobioréacteurs situés derrière ou dans le bâtiment.

Malheureusement, ces systèmes sont très coûteux, difficiles à mettre en œuvre. En particulier, ces systèmes ne permettent pas de diminuer significativement la consommation d'énergie fossiles, ni ne permettent d'obtenir des systèmes avec un rendement/autonomie comparable/similaire à ceux obtenues avec des dispositifs utilisant l'énergie fossile. Pour les systèmes avec des plantes, il y a aussi les problèmes du renouvellement et de l'entretien des plantes, qui sont compliqués, demandent beaucoup de mains d'oeuvre et sont difficilement automatisable. Ces problèmes sont prépondérants, par exemple pour les photobioréacteurs (PBR). En particuliers, les deux obstacles majeurs au développement des PBR traditionnels tiennent à :
- leur coût d'investissement, lié notamment au cout des produits verrier, à la complexité de la plomberie, pas toujours optimisée, qui doit arriver sous l'ouvrage et aux couteux ouvrages de serrurerie nécessaire a la stabilité.
- le coût de la régulation thermique des cultures de microalgues et/ou microorganismes, nécessitant une température maintenue généralement entre 15 et 28°C pour assurer une productivité adéquate.

Il existe donc un réel besoin d'un système palliant ces défauts, inconvénients et obstacles de l'art antérieur.

### Exposé de l'invention

La présente invention a notamment pour objet un module (D) photobioréacteur (PBR) d'isolation et/ou de régulation thermique d'un bâtiment, ledit module (D) comprenant un contenant de culture d'algues et/ou de microorganismes en milieu aqueux, au moins deux parois parallèles ou sensiblement parallèles définissant au moins un espace intérieur, ledit au moins un espace intérieur comprenant ledit contenant de culture d'algues et/ou de microorganisme en milieu aqueux, l'une de ces parois pouvant servir de paroi de façade ou être apposée à une paroi de façade du bâtiment, lesdites au moins deux parois étant des parois multicouches, lesdites au moins deux parois multicouches définissant une ou plusieurs lames d'air.

Ainsi, la présente invention a également pour objet une façade de bâtiment comprenant au moins un module (D) photobioréacteur (PBR) d'isolation et/ou de régulation thermique , c'est-à-dire un ou plusieurs modules (D) photobioréacteur (PBR) d'isolation et/ou de régulation thermique, identiques ou différents.

Le dispositif selon l'invention est dénommé « Photo-Bio-Reacteur-Rideau » ou (PBR).

Un des objectifs de la présente invention est de réaliser une double façade ventilée permettant la culture de microalgues en optimisant les apports solaires et la régulation thermique destinée à l'application de bâtiments.

Un autre objectif de la présente invention est de réaliser une simple façade ou double façade ventilée, permettant la culture, par exemple d'algue et/ou microalgues et/ou de microorganismes en optimisant les apports solaires et la régulation thermique, et destinée à l'application de bâtiments.

L'invention réside notamment dans le caractère intégrable du produit ou dispositif, associé à l'intérêt thermique et de maintenance d'une simple ou double façade. Au lieu d'installer des photobioréacteurs isolés derrière une façade, ou dans une serre ou sur une façade, il est possible d'utiliser grâce à la présente invention directement le photobioréacteur (PBR) ou « contenant de culture d'algues et/ou de microorganismes ». comme élément de façade

L'invention présente également un intérêt pour la maintenance d'une simple ou double façade telle que définie ci-dessous.

Grâce à la présente invention, on réalise ainsi une économie en mutualisant les éléments constructifs.

En outre, grâce à la présente invention, les procédés et filières industriels développés pour les triples façades ventilées, son réutilisés et mutualisés en permettant ainsi de réaliser un PBR « standardisé » à moindre coût.

Un des obstacles au développement des PBR traditionnels tient à leur coût d'investissement et notamment au coût des produits verrier, à la complexité de la plomberie qui doit arriver sous l'ouvrage et aux coûteux ouvrages de serrurerie nécessaires à la stabilité de l'édifice.

La présente invention répond aux besoins et inconvénients précités de l'art antérieur en intégrant le PBR au bâtiment.

La présente invention permet avantageusement une économie de place, de temps lors de la construction d'un bâtiment et une économie relative à la fabrication des PBR en mutualisant les éléments constructifs.

En outre avantageusement, on se sert du bâtiment pour assurer la stabilité des PBR ou modules PBR. Il n'y a donc plus besoin de serrurerie.

Dans la présente, on peut utiliser le vitrage pour réaliser la façade.

En outre, en rendant possible un déplacement latérale de la position d'arrivée des réseaux, notamment des circuits d'alimentation en eau et/ou milieu de culture on résout un problème de croisement de réseaux (problème de synthèse) et donc d'encombrement des réseaux qui s'en trouve optimisé.

Par une alternance astucieuse des modules vitrés transparents (pour éclairer les locaux du bâtiment) et des modules PBR selon l'invention (pour cultiver les algues et/ou microorganismes) on résout également et à coût réduit, les problèmes d'entrées et de sorties des réseaux, notamment des circuits d'alimentation en eau, en milieu de culture, en air comprimé, ainsi que des circuits de récolte/ de récupération de la biomasse, par exemple les algues, en résolvant les problèmes de « synthèse » et de croisements des réseaux dans l'épaisseur du plancher supportant le module mis en place sur la façade du bâtiment.

En d'autres termes, par une alternance astucieuse de modules (E), par exemple vitrés transparents (pour éclairer les locaux du bâtiment), et de module (D) photobioréacteur (PBR) d'isolation et/ou de régulation thermique d'un bâtiment selon l'invention (pour cultiver des algues, microalgues et/ou microorganismes), la présente invention permet de résoudre élégamment et à simple coût, les problèmes d'entrées et de sorties des réseaux, en résolvant les problèmes de « synthèse » et de croisements des réseaux dans l'épaisseur d'un faux-plancher. Là ou le photobioréacteur classique fait ses entrées de réseaux par le dessous.

La présente invention permet un assemblage horizontal, vertical ou mixte des modules (D) photobioréacteur (PBR) d'isolation et/ou de régulation thermique d'un bâtiment selon l'invention et permet de réaliser ainsi un grand photobioréacteur en réseau sur tout le long de ou sur la façade du bâtiment.

Selon l'invention, le module (D) comprend au moins deux parois parallèles, ou de préférence sensiblement parallèles, définissant au moins un espace intérieur, l'une de ces parois pouvant servir de paroi de façade du bâtiment ou être apposée à une paroi de façade d'un bâtiment. Avantageusement, les parois du module (D) peuvent être constituées de tout matériau connu de l'homme du métier. Ces parois peuvent être identiques ou différentes, par leur forme et/ou par le matériau qui les constitue. Il peut s'agir, par exemple de parois transparentes ou opaques. Lorsqu'il s'agit de parois transparentes, elles peuvent être par exemple en verre, en polycarbonate, en éthylène tétrafluoroéthylène (ETFE), en résine transparente, etc....Lorsqu'il s'agit de parois opaques, elles peuvent être par exemple en polycarbonate, en métal, par exemple inox, acier, aluminium.

Avantageusement, l'épaisseur des parois peut être indépendamment de 0,5 mm à 50 mm, par exemple de 6 à 24 mm.

Avantageusement, l'espacement des parois peut être indépendamment de 0,5 mm à 200 mm, par exemple de 40 mm à 100 mm.

Dans la présente, les parois décrites du module (D) peuvent être indépendamment des parois mono ou multicouches, par exemple, il peut s'agir d'une paroi constituée de 1 à 10 couches, par exemple de 1 à 3 couches.

Selon l'invention, les parois du module (D) sont indépendamment des parois multicouches, lesdites au moins deux parois multicouches définissant une ou plusieurs lames d'air.

Avantageusement, lorsque la paroi est multicouche, les couches peuvent être indépendamment séparées par un espace, par exemple un espace étanche comprenant, par exemple un gaz, par exemple de l'oxygène, de l'argon, de l'azote. Selon l'invention, au moins un espace étanche peut également constituer un contenant de culture pour les algues et/ou microorganismes.

Avantageusement, au moins un espace du module (D) peut être un espace ventilé, par exemple, via un flux d'air, par exemple un flux d'air laminaire, définissant ainsi une lame d'air ventilée.

Avantageusement, au moins un espace du module (D) peut être un espace respirant, par exemple, via un joint poreux, pouvant ainsi définir une lame d'air respirante, permettant avantageusement d'éviter la formation de condensation, par exemple sur les parois du module.

Selon l'invention, au moins un espace intérieur du module (D) comprend un contenant de culture de culture d'algues et/ou de microorganismes
Avantageusement, chaque paroi du module (D) peut comprendre indépendamment, par exemple, un simple (M₁), double (M₂) ou triple (M₃) vitrage, un vitrage multi-parois, ou encore un assemblage de ces différents vitrages.

Ces parois définissent avantageusement un espace intérieur thermiquement contrôlé, en particulier un espace dont la température peut être intermédiaire et/ou régulée entre l'extérieur et l'intérieur du bâtiment, favorable ou permissif à la croissance des microalgues et/ou microorganismes.

Avantageusement, au moins un des espaces définis par le simple (M₁), double (M₂) ou triple (M₃) vitrage ou le vitrage multi-parois peut être ou comprendre un contenant de culture au sens de la présente invention.

Avantageusement, les inventeurs ont démontré de manière surprenante que lorsque les parois du module (D) sont des parois multicouches, elles permettent avantageusement de définir une ou plusieurs lames d'air permettant :
- d'isoler thermiquement les contenants / milieu de cultures d'algues et/ou microorganismes de l'extérieur en hiver par une lame d'air isolante,
- d'éviter la formation de condensation, qui serait néfaste à la productivité des microalgues et/ou microorganismes en diminuant l'énergie lumineuse, grâce à une lame d'air respirante (L₁),
- de favoriser la ventilation naturelle des cultures de microalgues et/ou microorganismes grâce à une lame d'air ventilée (L₂) par un flux d'air, ce flux d'air (L) étant en échange, l'été, avec l'extérieur, et l'hiver, avec l'intérieur du bâtiment et/ou de la coursive (S) dans le cas d'une double façade.

Avantageusement, lorsque l'espace intérieur du module (D) comprend un contenant de culture, les surfaces externes des parois contenant de culture peuvent être en contact direct ou non avec la surface interne des parois du module (D).
Avantageusement, le module (D) peut comprendre un espace et/ou un renfoncement dans lequel peut être placé ou logé un éclairage et/ou rétro-éclairage. Par exemple, le contenant peut avoir une forme, en coupe transversale, de haricot, la lumière pouvant être logée dans le creux du haricot. Des creux multiples, ou ondulations, peuvent être prévues pour loger un éclairage artificiel permettant de fournir aux algues et/ou microorganismes cultivés la lumière nécessaire à leur croissance. Il peut s'agir également d'un éclairage artificiel.

Avantageusement, selon l'invention lorsque les parois du module (D) définissent un espace étanche pouvant comprendre ou constituer le contenant de culture de microalgues et/ou microorganismes, elles peuvent assurer également, côté extérieur du bâtiment, l'éclairage des algues et/ou microorganismes, par exemple via leur transparence, et côté bâtiment les échanges thermiques avec soit l'intérieur du bâtiment, dans le cas d'une simple façade, soit l'espace intérieur de la double-façade (S), dans le cas d'une double façade.

Les parois du module (D) peuvent être des parois multicouches dont une ou plusieurs est située(s) entre l'extérieur et le milieu de culture d'algues, de microalgues et/ou microorganismes. Cette / Ces une ou plusieurs couche(s) permet(tent) avantageusement d'assurer l'éclairage naturel des cultures/ milieu de culture d'algues, de microalgues et/ou microorganismes.

La présente invention a également pour objet l'utilisation d'un module (D) photobioréacteur (PBR) d'isolation et/ou de régulation thermique d'un bâtiment selon l'invention pour la fabrication d'un bâtiment.

Aventageusement un dispositif d'isolation de bâtiment peut comprendre au moins un module (D) photobioréacteur (PBR) d'isolation et/ou de régulation thermique tel que défini ci-dessus, et au moins un module (E).

Avantageusement, le module (E) peut comprendre au moins deux parois parallèles, ou de préférence sensiblement parallèles, définissant au moins un espace intérieur, l'une de ces parois pouvant servir de paroi de façade du bâtiment ou être apposée à une paroi de façade d'un bâtiment.

Avantageusement, les parois du module (E) peuvent être constituées de tout matériau connu de l'homme du métier. Ces parois peuvent être identiques ou différentes, par leur forme et/ou par le matériau qui les constitue. Il peut s'agir, par exemple de parois transparentes ou opaques. Lorsqu'il s'agit de parois transparentes, elles peuvent être par exemple en verre, en polycarbonate, en éthylène tétrafluoroéthylène (ETFE), en résine transparente, etc..... Lorsqu'il s'agit de parois opaques, elles peuvent être par exemple en béton, en bois, en métal, par exemple inox, acier, aluminium.

Avantageusement, l'épaisseur des parois peut être de 0,5 mm à 100 mm, par exemple de 5 mm à 50 mm,

Avantageusement, l'espacement des parois peut être indépendamment de 0,5 mm à 200 mm, par exemple de 5 mm à 50 mm.

Avantageusement, le module (E) peut être par exemple un simple, double ou triple vitrage, ou encore un vitrage dit « multi-parois », ce dernier cas étant défini par exemple par l'assemblage de plusieurs verres via le profilé de filière, ou de toute autre technique mixte connu de l'homme du métier.

Avantageusement, l'espace interne du module (E) peut être un espace étanche ou perméable.

Avantageusement, lorsque l'espace interne du module (E) est étanche, il peut comprendre un gaz, par exemple de l'air, de l'argon.

Avantageusement, lorsque l'espace interne du module (E) est perméable, le module (E) peut comprendre à au moins une de ces extrémités au moins une ouverture, un système de ventilation, un orifice. Avantageusement, lorsque le module (E) est perméable, il peut permettre la circulation de fluides, par exemple de gaz et/ou de liquide dans le module (E). Avantageusement, la circulation de fluides dans le module (E) peut permettre un échange thermique entre l'extérieur et l'intérieur du bâtiment permettant avantageusement une régulation de la température du bâtiment.

Avantageusement, le module (E) peut être monté sur des ossatures, par exemple des ossatures métalliques, par exemple en aluminium, des ossatures en bois, ou des ossatures en plastique polymérique, par exemple en polychlorure de vinyle

La présente invention a également pour objet une façade de bâtiment, également nommé simple façade et/ou une façade extérieure (A) d'une façade ou d'une double façade, comprenant au moins un module (D) photobioréacteur (PBR) d'isolation et/ou de régulation thermique , avantageusement comprenant au moins un module (D) photobioréacteur (PBR) d'isolation et/ou de régulation thermique et au moins un module (E) tel que défini ci-dessus.

Selon l'invention, par simple façade on entend une façade de bâtiment comprenant la surface extérieure du bâtiment (A').

Selon l'invention, par double façade on entend une façade de bâtiment comprenant la surface du bâtiment également nommé une façade extérieure (A), et une surface extérieure ou intérieur au bâtiment (N ou B), c'est à dire une surface autre que la surface extérieure du bâtiment, par exemple une enveloppe extérieure et/ou une surface comprise dans le bâtiment, définissant une double peau ou espace tampon.

Avantageusement, la surface extérieure et/ou intérieur au bâtiment (N ou B) peut être transparente et peut comprendre des volets d'aérations, ladite double peau étant de préférence fixe et parallèle à une façade du bâtiment. L'enveloppe extérieure de la double peau peut être par exemple une surface vitrée, une surface en ETFE, une surface constituée de menuiserie et vitrage, de menuiserie et de résilles en acier, en métal, en inox, en galva, en nylon ou carbone. Les volets d'aération permettent d'aérer l'espace entre l'enveloppe extérieure de la double peau ou double paroi et la façade du bâtiment.

Avantageusement, l'enveloppe intérieure de la double façade est de préférence étanche, de préférence, elle peut permettre de limiter les passages d'air et/ou courants d'air, notamment en hiver, afin de protéger le contenant de culture des algues des variations de température.

Avantageusement, la double façade permet avantageusement de former un matelas d'air autour du bâtiment et/ou de confiner de la chaleur et/ou la fraicheur émise par le bâtiment, et ainsi permettre d'utiliser la chaleur et/ou la fraicheur émise par le bâtiment pour chauffer et/ou rafraichir la culture d'algues et/ou de microorganismes.

Avantageusement, la double façade permet en outre de stocker les calories du bâtiment, d'améliorer l'inertie calorique d'un bâtiment et de réguler la température de la culture.

Avantageusement, une façade et/ou une façade extérieure (A) d'une façade ou d'une double façade peut comprendre deux types de modules:
- un module (E) assurant la vue et l'éclairement,
- un module D servant à la fois d'éléments de façade et de photobioréacteurs (PBR).

Avantageusement et de manière surprenante, la présente, par une conception spécifique/particulière des modules (E) et (D) de mur-rideaux, permet d'assurer une régulation thermique des cultures de microalgues et/ou microorganismes :
- passive par ventilation naturelle de la façade et échanges thermiques soit avec l'air extérieur l'été, soit avec l'air intérieur du bâtiment ou de l'espace de la double-façade l'hiver,
- et/ou active par échange thermique avec les circuits d'eau du bâtiment.

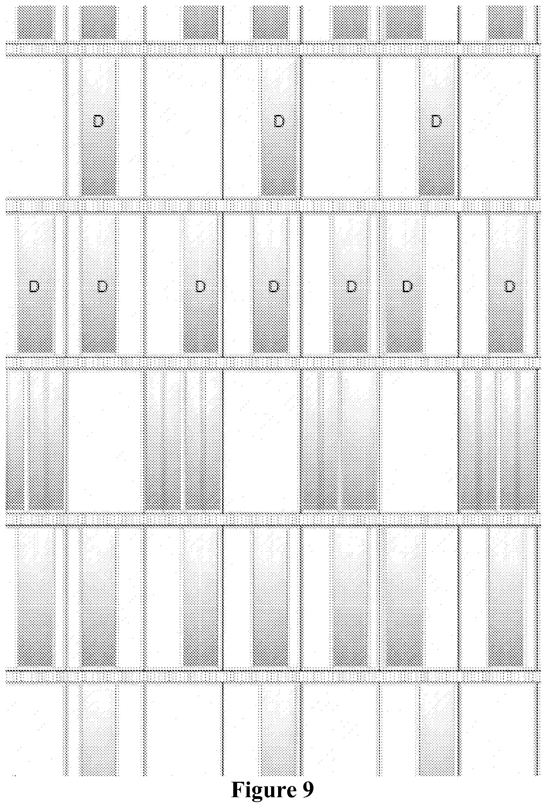

Avantageusement, la disposition des modules (D) et (E) sur la façade et/ou une façade extérieure (A) d'une façade ou d'une double façade peut être en quinconce, en alternance, par exemple un sur deux, un sur trois, horizontalement et/ou verticalement. Ainsi, la disposition des modules sur la façade, par exemple une façade extérieure (A), par exemple d'une double façade, peut être en damier, linéaire 1 sur 2, 1 sur 3, 1 sur 4, en diagonale 1 sur 2, 1 sur 3, 1 sur 4, ou tout autre calepinage adapté à l'éclairage naturel et à la régulation thermique du bâtiment et des algues (voir figure 9).
Avantageusement, le module (D) et/ou (E) peut comprendre indépendamment sur au moins une de leurs surfaces externes un habillage et/ou un système de protection. Il peut s'agir par exemple d'une plaque métallique, par exemple en aluminium, en zinc. En outre, l'habillage ou un système de protection peut être disposé directement sur au moins une surface externe du module (D) et/ou (E). Selon l'invention, l'habillage ou un système de protection peut être disposé sur la surface du module située à l'intérieur du bâtiment

Avantageusement, la hauteur du module (D) et/ou (E) peut-être indépendamment comprise par exemple entre 1 et 10 m, de préférence entre 2 et 3,60 m. En fait, toute hauteur peut être utilisée, pourvu qu'elle soit pratique pour la construction du bâtiment.

Avantageusement, le module (E) et/ou (D) peu(ven)t être préfabriqué(s), par exemple il peut s'agir de modules fabriqués en usine prêt pour mise en œuvre, par exemple pour la construction d'une façade et/ou d'une façade extérieure (A) d'une double façade.

Avantageusement, l'assemblage des modules (D) et (E) peut être, par exemple, réalisé par fixation desdits modules sur une ossature métallique (C), directement sur le bâtiment ou sur le nez des dalles de planchers du bâtiment, par exemple via des raidisseurs ou aiguilles (F).

Avantageusement, les modules (D) et (E) assemblés peuvent constituer la façade d'un bâtiment (B) et/ou la façade extérieure (A) d'une façade ou d'une double façade.

Avantageusement la présente invention peut également comprendre deux façades dont l'une, à l'extérieur va alterner deux types d'ensembles vitres préfabriqués :
- l'un à double vitrage assure la vue et l'éclairement des locaux. Il intègre la ventilation naturelle de la double façade en partie haute et en partie basse.
- l'autre comprend un triple vitrage composé d'un double vitrage et d'un simple séparé par un espace étanche, apte à recevoir et intégrer des contenants de culture étanches servant à la fois d'éléments de façade et de photobioréacteurs (PBR).

Ces deux façades définissent avantageusement un espace thermiquement contrôlé d'une température intermédiaire entre l'extérieur et l'intérieur favorable à la croissance et à la culture de microalgues.

L'invention réside notamment dans le caractère intégré de l'ensemble « façade + Photobioréacteur » dans un ensemble ou module plurivalent.

Une première façade, A extérieure, étanche à l'eau et à l'air en hiver, étanche à l'eau en été.

La façade A est un mur rideau composé de modules vitrés D et E assemblés et fixés sur une ossature métallique en aluminium ou directement sur le nez de dalle. Les raidisseurs ou aiguilles (F) éventuels pourront être en aluminium, ou en métal, ou en Befup ou en bois.

Les modules D forment des ensembles préfabriqués étanches montés sur ossatures F métalliques, ou en aluminium, à rupteur de pont thermique. Ils servent à la fois d'élément constituant de la façade et de Photo bio réacteurs.

Une deuxième façade, (N) intérieure, est composée de châssis fixe ou de mur rideau toute hauteur en aluminium Le vitrage du mur rideau pourra être a simple vitrage ou a double vitrage.

Le contenant de culture d'algues et/ou de microorganismes peut par exemple avoir une épaisseur comprise entre 5 et 20 centimètre, par exemple de 8 centimètres.

Avantageusement, un module supplémentaire (X) côté bâtiment peut également être disposé, par exemple au contact d'une des surfaces externes du modules (D) précité.

Avantageusement, le module (X) peut être composé de plusieurs parois définissant un espace intérieur. Il peut s'agir par exemple d'un panneau creux, par exemple un panneau comprenant au moins une cavité, par exemple un panneau solaire thermique.

Avantageusement, la paroi du module (X) peut être dans tout matériau ayant une bonne conductivité thermique, il peut s'agir, par exemple de parois en métal, par exemple en inox, en acier,

Avantageusement, l'espace intérieur du module (X) peut comprendre un gaz ou liquide, par exemple de l'eau, ou un liquide caloporteur antigel, anti corrosion, bactéricide, fongicide, anti tartre, à base de mono propylène glycol

Avantageusement, l'eau peut être, par exemple une eau provenant d'un bâtiment, par exemple du bâtiment où se trouve le module (X). Par exemple, il peut s'agir d'eau provenant du circuit d'eau courante du bâtiment, ou bien du circuit d'eaux grises du bâtiment.

Avantageusement, le module (X) peut également permettre d'assurer des échanges thermiques entre des flux d'eau du bâtiment, par exemple en intégrant directement le passage de ces flux et/ou en intégrant le passage de flux caloporteurs en échange thermique avec les flux d'eau du bâtiment et le PBR ou module (D).

Avantageusement, l'invention permet que les algues et/ou microorganismes compris dans le PBR bénéficient de l'ambiance thermique contrôlée :
- de l'intérieur du bâtiment, lorsque les modules D et E constituent la façade du bâtiment (A'),
- de l'espace intermédiaire (S) de la double façade lorsqu'ils constituent la façade extérieure (A) de la double-façade

Elles bénéficient par ailleurs des échanges thermiques possibles avec les circuits d'eau du bâtiment.

Les modules D et E permettent avantageusement, par ailleurs d'assurer l'étanchéité du bâtiment vis-à-vis de l'air, en particulier du froid, de l'eau et du vent l'hiver et de l'eau et de la chaleur l'été.

Avantageusement, le contenant de culture d'algues et/ou de microorganismes, notamment inclus dans le module (D), peut incorporer une pièce moulée.

La pièce moulée peut être dans toute matière permettant d'être incorporée dans le contenant de culture d'algues et/ou de microorganismes. Par exemple la pièce moulée peut être en résine.

La pièce moulée peut avantageusement comprendre un ou plusieurs tuyaux permettant d'alimenter les algues et/ou les microorganismes en gaz, en eau, en nutriments, etc.

De préférence, le ou les tuyaux de la pièce moulée sont intégrés dans ladite pièce moulée. En d'autres termes, le ou les tuyaux de la pièce moulée font partie intégrante de la pièce moulée.

Le ou les tuyaux de la pièce moulée peuvent par exemple être reliés à des tuyaux situés par exemple dans un faux-plafond ou un faux-plancher, afin d'être reliés à des réservoirs.

Le ou les tuyaux de la pièce moulée peuvent être disposés sur la partie inférieure de la pièce moulée, c'est-à-dire la partie la plus proche du sol. Cela permet de laisser la partie supérieure du contenant de culture d'algues et/ou de microorganismes disponible pour une intervention mécanique. Par exemple une trappe peut être située sur la partie supérieure du contenant de culture d'algues et/ou de microorganismes.

Avantageusement, au moins une pièce spécifique ou pièces moulé, par exemple 1 à 3 pièces spécifiques (G₁, G₂, G₃) peu(ven)t également être incorporée(s) dans le contenant de culture d'algues et/ou de microorganismes et/ou le module (D) afin d'assurer la connectique et/ou la dynamique des différents fluides.

Avantageusement, la pièce spécifique peut-être composée de tout matériau connu de l'homme du métier :
- permettant d'être incorporée dans le contenant de culture d'algues et/ou de microorganismes ; et/ou
- compatible avec des cultures d'algues et/ou de microorganismes.

Il peut s'agir, par exemple, d'un matériau choisi parmi l'inox, du plastique, du caoutchouc, de la résine.

Avantageusement, la ou les plusieurs pièce(s) spécifique(s) peut être avantageusement moulée.

La pièce spécifique peut avantageusement comprendre un ou plusieurs tuyaux permettant d'alimenter le milieu de culture des algues et/ou des microorganismes, par exemple avec en gaz (K), en eau, en nutriments, etc. (I) ou d'extraire le milieu algal (J) et/ou le milieu de culture.

Avantageusement, le ou les tuyaux de la ou lesdites pièce(s) spécifique(s) peu(vent)t être avantageusement intégrés dans ladite pièce spécifique. Par exemple, le ou les tuyaux de la pièce spécifique peu(ven)t faire partie intégrante de ladite pièce spécifique.

Avantageusement, le ou les tuyau(x) de la ou plusieurs pièce(s) spécifique(s) peu(ven)t, par exemple, être reliés à des tuyaux situés par exemple dans un faux-plafond (Q) ou un faux-plancher (T).

Avantageusement, le ou les tuyaux de la pièce spécifique peu(ven)t être disposés sur la partie inférieure de la pièce, c'est-à-dire la partie la plus proche du sol. Cette disposition permet avantageusement de laisser la partie supérieure du contenant de culture d'algues et/ou de microorganismes disponible pour une intervention mécanique. Par exemple une trappe peut être située sur la partie supérieure du module (D) et/ou du contenant de culture d'algues et/ou de microorganismes.

Avantageusement, au moins un orifice et/ou tuyau faisant face au contenant de culture et/ou module (D) peut être orienté selon un angle compris de 0 à 90 degrés par rapport à l'axe vertical du contenant de culture et/ou module (D). Avantageusement, l'orientation dudit au moins un orifice et/ou tuyau permet de générer, de part l'injection de fluides ou de gaz, une agitation dans le milieu de culture évitant ainsi la formation de dépôt d'algue dans le contenant et/ou module (D).

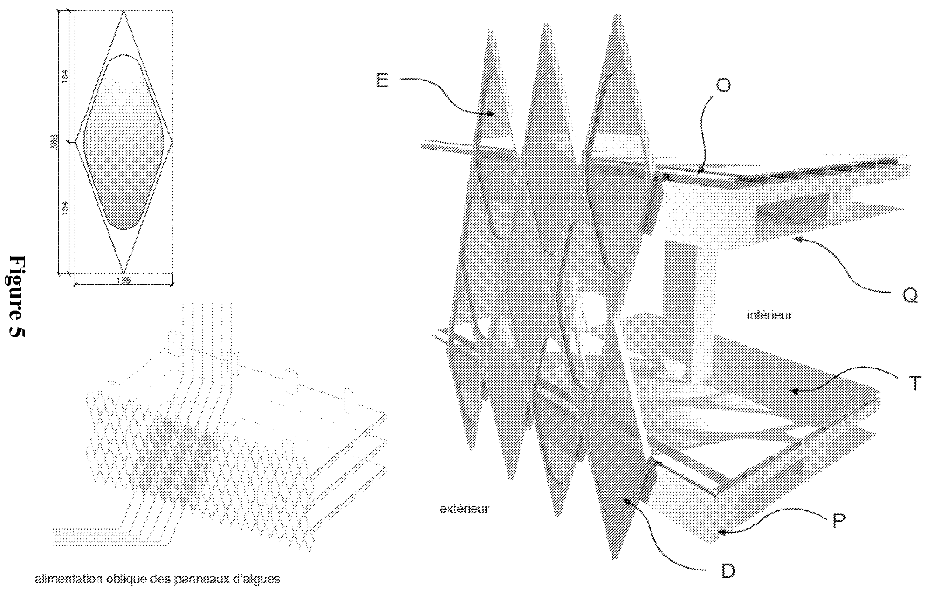

Le contenant de culture d'algues et/ou de microorganismes peut avantageusement être sous la forme d'un losange dont l'un des sommets est situé vers le bas, c'est à dire vers le sol, par exemple comme montré à la figure 5.

Le contenant de culture d'algues et/ou de microorganismes peut constituer tout ou partie d'une façade de bâtiment. Par exemple, lorsque plusieurs contenants de culture d'algues et/ou de microorganismes composent la surface d'un bâtiment, notamment dans le module (D), ils peuvent représenter 5 à 100% de la surface du bâtiment, de 5 à 50 % de la surface du bâtiment, de 50 à 100% de la surface du bâtiment

Lorsque plusieurs contenants de culture d'algues et/ou de microorganismes composent la surface d'un bâtiment, ils peuvent par exemple être disposés de façon alternée avec les vitres du bâtiment. Par exemple il peut s'agir d'une alternance verticale et/ou d'une d'alternance horizontale.

Les modules (D) et (E), ainsi que le contenant de culture d'algues et/ou de microorganismes permet avantageusement d'isoler le bâtiment et de le rendre étanche à l'air et à l'eau.
Le module (D) photobioréacteur (PBR) d'isolation et/ou de régulation thermique (D) d'un bâtiment selon l'invention peut être nettoyé de différentes façons. Le nettoyage permet notamment d'éliminer les algues et/ou microorganismes qui se sont déposés à la surface interne du contenant de culture d'algues et/ou de microorganismes.

Par exemple, le module (D) photobioréacteur (PBR) d'isolation et/ou de régulation thermique d'un bâtiment selon l'invention peut être nettoyé par ultraviolets. Les ultraviolets peuvent par exemple être appliqués depuis l'extérieur du contenant de culture d'algues et/ou de microorganismes.

Les ultraviolets peuvent être appliqués par tout dispositif connu de l'homme du métier permettant d'émettre des ultraviolets.
Le module (D) photobioréacteur (PBR) d'isolation et/ou de régulation thermique d'un bâtiment selon l'invention peut également être nettoyé par ultrasons. Les ultrasons peuvent par exemple être appliqués depuis l'extérieur du contenant de culture d'algues et/ou de microorganismes.
Le module (D) photobioréacteur (PBR) d'isolation et/ou de régulation thermique d'un bâtiment selon l'invention peut également être nettoyé par un système mécanique embarqué. Le système mécanique embarqué peut être appliqué depuis l'intérieur du contenant de culture d'algues et/ou de microorganismes. Par exemple, le système mécanique embarqué peut être introduit dans le contenant de culture d'algues et/ou de microorganismes depuis une trappe situé sur la partie supérieure du contenant de culture d'algues et/ou de microorganismes.

Par exemple le système mécanique embarqué peut être un rouleau, par exemple un rouleau flottant.

Le moyen de nettoyage peut être également de l'oxygène liquide ou de l'ozone dissout dans de l'eau.

Les moyens de nettoyage décrits ci-dessus permettent avantageusement de nettoyer les deux faces internes du contenant de culture d'algues et/ou de microorganismes.

Les moyens de nettoyage décrits ci-dessus peuvent avantageusement être automatisables. Par exemple, le moyen d'application d'ultraviolet et/ou le moyen d'application d'ultrasons peuvent être disposés sur un rail permettant de nettoyer plusieurs contenants de culture d'algues et/ou de microorganismes. Lorsque le bâtiment possède plusieurs étages, il peut y avoir par exemple un rail par étage ou un rail pour plusieurs étages.

Par ailleurs, la surface du bâtiment peut comprendre des clapets de ventilations. Par exemple les clapets de ventilation peuvent être situés à la base des modules (D) et/ou (E) et/ou des contenants de culture d'algues et/ou de microorganismes.

En outre, la surface du bâtiment peut également comprendre des clapets de ventilations qui peuvent être situés à la base et/ou au sommet des modules (D) et/ou (E).
Le module (D) photobioréacteur (PBR) d'isolation et/ou de régulation thermique d'un bâtiment selon l'invention peut également comprendre et/ou être connecté à un ou plusieurs des éléments suivants
- un moyen d'alimentation de la culture d'algue et/ou de microorganisme
- un moyen d'injection dans la culture d'algues et/ou de microorganisme d'un effluent, ledit effluent provenant d'un bâtiment,
- un moyen de régulation de la température de la culture d'algue,
- un moyen de connexion au bâtiment duquel provient l'effluent audit moyen d'injection, ledit moyen ayant pour fonction de conduire l'effluent du bâtiment jusqu'au moyen d'injection, et
- éventuellement un éclairage favorable à la culture d'algues et/ou de microorganisme.

Selon l'invention, par « bâtiment » on entend toute construction servant à loger des hommes, des animaux ou des choses. Il peut s'agir par exemple d'une construction, d'un bâtiment industriel et/ou de bureaux et/ou d'habitation et/ou agricole, par exemple une maison, un immeuble, une centrale thermique et/ou d'un ouvrage d'art souterrain, par exemple une infrastructure souterraine de circulation automobiles et/ou ferroviaire, par exemple un tunnel autoroutier, un tunnel de métro, un parking, un tunnel, une voierie souterraine, un espace sous dalles, une caverne ou grotte aménagée en habitat humain, animal, de culture ou pour utilisation industrielle ou de stockage.

Avantageusement, l'effluent peut être, par exemple, un effluent gazeux ou un effluent liquide.

Avantageusement, par « effluent liquide » on entend un effluent seul ou un mélange d'effluents liquides. Il peut s'agir, par exemple de tout liquide et/ou solution vicié issu d'un bâtiment. L'effluent liquide peut être vicié par l'occupation humaine. Il peut s'agir, par exemple, d'eaux usées issues des sanitaires, d'un liquide contenant un contaminant par exemple un métal, par exemple du plomb, du nickel, une substance polluante, par exemple des nitrates, des sels.

La présente invention, par exemple via le module (D) photobioréacteur (PBR) d'isolation et/ou de régulation thermique, permet notamment de retraiter les effluents liquides pollués, par exemple ceux précités, par exemple aussi des effluents avec des métaux, sels, composés chimiques, et autres polluants qui peuvent être, par exemple, évacués des bâtiments.

Avantageusement, le traitement de l'effluent liquide un ou du mélange d'effluents liquides peut consister, par exemple, à éliminer, c'est-à-dire extraire, de l'effluent liquide au moins un polluants, un contaminant par exemple un métal, par exemple du plomb, du nickel, nitrates, sels et toute substance polluante. Il peut par exemple consister à désaliniser un effluent liquide et/ou à renouveler le liquide traité. Le traitement peut être fonction de l'algue et/ou du microorganisme choisie. Selon l'invention, l'algue et / ou le microorganisme peut être choisi en fonction du traitement souhaité de l'effluent à traiter.

Avantageusement, par « effluent gazeux », on entend un seul effluent gazeux ou un mélange d'effluents gazeux. Il peut s'agir de tout air vicié issu d'un bâtiment ou de tout mélange d'air vicié issu d'un bâtiment. L'effluent gazeux peut être de l'air vicié par l'occupation humaine, par la circulation de véhicules dans le bâtiment, par exemple d'un parking ou de plusieurs parking, ou aux abords des bâtiments, par exemple tunnels, voieries souterraines, espaces sous dalles, par les productions industrielles, de l'air issus des moyens de chauffage du bâtiment, notamment au gasoil ou au gaz. Selon l'invention, effluent gazeux peut être un gaz comprenant par exemple du CO₂, du dioxyde d'azote ou de l'air viciés par l'occupation humaine, par la circulation de véhicules dans le bâtiment (parkings), par la circulation ferroviaire, ou aux abords des bâtiments (tunnels, voieries souterraines, espaces sous dalles), par les productions industrielles.

La présente invention permet notamment de retraiter les effluents gazeux pollués en CO₂ et dioxydes d'azote, monoxyde de carbone, et autres polluants qui sont évacués des bâtiments fabriqués par les humains, notamment ceux précités, notamment par les centrales de ventilations et autres exemples cités ci-dessous.

Avantageusement, le traitement de l'effluent gazeux peut consister par exemple à éliminer, c'est-à-dire extraire, de l'effluent gazeux du CO₂ et/ou du NO₂. Avantageusement, les algues transforment le CO₂ et/ou le NO₂ en oxygène qui peut permettre, par exemple, de renouveler l'atmosphère du bâtiment ou être évacué. Il peut s'agir d'autres gaz que ceux précités, et il peut s'agir également de particules et poussières présentes dans ces gaz. Le traitement peut être fonction de l'algue et/ou du microorganisme choisie. De la même manière, l'algue et/ou le microorganisme peut être choisie en fonction de l'effluent à traiter.

Avantageusement, le moyen d'injection de l'effluent dans la culture d'algues et/ou microorganisme peut être connecté à un moyen de récupération de l'effluent à traiter permettant de récupérer le ou les effluents issus d'un ou de plusieurs bâtiment(s) et/ou d'un ou plusieurs ouvrage(s) d'art souterrain(s), par exemple une infrastructure souterraine de circulation automobile, ferroviaire, par exemple un tunnel autoroutier, un tunnel de métro pour les injecter dans la culture d'algues. Avantageusement la présente invention permet de traiter l'air vicié ou le liquide vicié d'un bâtiment en y connectant le dispositif précité utilisé dans la présente invention

Avantageusement, le moyen de récupération dudit effluent peut être choisi dans le groupe comprenant un ventilateur, une pompe aspirante, un circuit d'aération, un circuit de climatisation, un circuit de filtration d'air d'un bâtiment. Tout moyen de récupération d'un effluent à traiter peut être utilisé. Ce moyen de récupération peut être par exemple un tuyau (« pipeline ») permettant de transporter le ou les effluent(s) issu(s), par exemple, d'un bâtiment ou de plusieurs bâtiments, par exemple d'une ou plusieurs centrale(s) thermique(s) ou tout autre bâtiment tel que ceux précités. Bien entendu, ce moyen de récupération est connecté au moyen d'injection afin d'apporter le l'effluent au contact des algues et/ou des microorganismes en culture, lesdites algues et/ou des microorganismes ayant pour fonction de métaboliser les éléments polluants et/ou indésirables de l'effluent, par exemple gazeux, par exemple le CO₂ et/ou du NO₂ pour les éliminer.

Selon l'invention, le contenant de culture d'algue et/ou de microorganisme peut être tout contenant connu de l'homme du métier. Il peut se présenter par exemple sous une forme choisie dans le groupe comprenant un tube, un cylindre, un tube plat, un tube ondulé sur sa longueur et/ou sa largeur, un panneau creux, une sphère, un cube, un parallélépipède rectangle, une spirale, parallélépipède rectangle avec des bords arrondis, une forme creuse sans arrête vive, d'un sachet. Dans le vocabulaire dans le domaine de l'architecture, le terme « tube » couvre d'ailleurs toutes ces structures possibles, dès lors qu'elles sont creuses. De préférence, selon l'invention, la forme creuse est sans arrête vive. Aussi, dans la présente, par « tube », on entend toute forme de contenant permettant de contenir une culture d'algue et/ou de microorganisme, y compris un tube ou un panneau creux. Par exemple, il peut s'agir d'un sachet ultramince, par exemple un sachet en éthylène tétrafluoroéthylène (ETFE) ; d'un verre profilé, par exemple de forme parallélépipède, de préférence avec des bords arrondis, un panneau creux en verre profilé de préférence permettant une culture d'algues et/ou de microorganismes. Ce contenant forme en fait un réacteur dans lequel l'algue et/ou de microorganismes est cultivée. Ainsi, toute forme appropriée à la culture d'algue convient.

Le contenant de culture d'algue et/ou de microorganismes peut par exemple se présenter sous une forme choisi dans le groupe comprenant un tube plat, un panneau creux, un parallélépipède rectangle, un parallélépipède rectangle avec bord arrondis et une forme creuse sans arrête vive.

De préférence, le contenant de culture d'algue et/ou de microorganismes se présente sous une forme choisi dans le groupe comprenant un tube plat, un panneau creux, un parallélépipède rectangle, un parallélépipède rectangle avec bord arrondis et une forme creuse sans arrête vive.

De préférence encore, la forme du contenant de culture d'algues et/ou de microorganismes est un parallélépipède rectangle creux avec des bords arrondis, par exemple un panneau creux avec des bords internes, et éventuellement externes, arrondis, ou un tube. Avantageusement, le dispositif de culture est sans arrête vive. En effet, l'absence d'arrête permet d'éviter l'accumulation et/ou l'accrochage des algues et/ou microorganismes qui est observée dans les contenants présentant des arrêtes vives, au niveau des creux formés par ces arrêtes.

Avantageusement, le contenant est de préférence un contenant transparent à la lumière. Il peut s'agir par exemple d'un contenant en verre profilé, d'un tube en polycarbonate, en plexiglas. Il s'agit bien sûr de la paroi du contenant. Ceci est particulièrement préféré lorsque l'algue ou le microorganisme en culture nécessite pour croître et/ou traiter l'effluent de la lumière, et que la lumière utilisée est la lumière naturelle.

Avantageusement, l'épaisseur du contenant, c'est-à-dire de sa paroi, peut être comprise entre 5 cm et 60 cm, de préférence entre 15 et 20 cm. L'épaisseur peut être de 5 mm à 60 mm, de 15 à 20 mm II peut s'agir en fait de toute épaisseur assurant la tenue du contenant lorsqu'il est rempli par le milieu de culture et les algues et ou microorganisme. L'homme du métier pourra aisément déterminer cette épaisseur.

Avantageusement, la hauteur du contenant peut-être comprise par exemple entre 1 et 10 m, de préférence entre 2 et 8,50 m. En fait, toute hauteur peut être utilisée, pourvu qu'elle soit constructible. En outre la hauteur du contenant peut être identique ou différente par rapport à la hauteur du module dans lequel il peut se trouver.

.Avantageusement, lorsque le contenant est horizontal, la longueur du contenant peut être, par exemple égale à la longueur du bâtiment et/ou à sa largeur, par exemple 100 mètres.

Lorsque le contenant est horizontal ou incliné, il peut être disposé sur le toit du bâtiment. L'inclinaison du toit peut commander l'inclinaison du contenant.

Avantageusement, le contenant peut comprendre un renfoncement dans lequel peut être placé ou logé un éclairage et/ou rétro-éclairage. Par exemple, le contenant peut avoir une forme, en coupe transversale, de haricot, la lumière pouvant être logée dans le creux du haricot. Des creux multiples, ou ondulations, peuvent être prévues pour loger un éclairage artificiel permettant de fournir aux algues et/ou microorganismes cultivés la lumière nécessaire à leur croissance et/ou au traitement de l'effluent. Il peut s'agir également d'un éclairage artificiel.

Avantageusement, lorsque le contenant de culture est un tube ou un panneau creux, le tube ou panneau creux est également, et pour les mêmes raisons que celle précitées, transparent à la lumière à laquelle est sensible l'algue et/ou le microorganisme pour sa culture. Cela permet avantageusement à l'algue et/ou au microorganisme de profiter de la lumière naturelle et/ou artificielle pour son métabolisme, en particulier la photosynthèse, notamment en vue de traiter l'effluent. Comme indiqué précédemment, il peut s'agir par exemple d'un tube ou panneau en verre, d'un tube ou panneau en polycarbonate ou en plexiglas, ou en tout autre matériau convenant à la mise en œuvre de la présente invention.

Avantageusement, lorsqu'il s'agit d'un tube, le diamètre extérieur du tube peut être compris par exemple entre 20 et 100 cm, de préférence entre 40 et 80 cm. L'épaisseur peut être par exemple celles précitées.

Avantageusement, la hauteur des tubes peut-être comprise par exemple entre 1 et 10 m, de préférence entre 2 et 8,50 m. Les mêmes remarques que celles-ci-dessus aux contenant en général sont applicables pour la hauteur.
Avantageusement, le contenant, par exemple le tube ou le panneau, peut être par exemple multicouche. Il peut comprendre par exemple, de l'extérieur vers l'intérieur, de manière concentrique, une couche extérieure, une couche médiane et une couche intérieure, et comprendre en outre un moyen d'éclairage ou rétroéclairage. Le rétroéclairage permet d'éclairer la culture, pour les raisons indiquées ci-dessus, par exemple lorsque l'environnement ne donne pas suffisamment de lumière ou que l'utilisateur souhaite stimuler la culture des algues et/ou microorganismes.

Par couche on entend l'espace crée entre 2 contenants, par exemple tubes ou panneau, concentriques, c'est-à-dire placés l'un dans l'autre l'axe des contenants, par exemple tubes ou panneaux, étant parallèle et laissant un espace entre les contenants, par exemple les tubes ou panneaux. Par concentrique on entend un ou plusieurs contenants, par exemple tube(s) ou panneau(x), placé(s) dans un ou plusieurs autre(s) tube(s).

La couche est donc délimitée par les parois des contenants, par exemple les tubes et/ou panneaux. Les contenants peuvent être identiques ou différents dans leur constitution, c'est-à-dire forme et matériaux utilisés. La distance entre les contenants concentriques créée un espace délimité par les parois des contenants. Cet espace est fonction du diamètre de chacun des contenants disposés concentriquement, de préférence dans le sens de la longueur.

Avantageusement, la couche du contenant située du côté du bâtiment peut avantageusement être une pièce emboutie, extrudée, étirée, resoudée, sertie ou composite.

De préférence, la surface du contenant de culture en contact avec le milieu de culture est une surface qui inhibe ou empêche toute adhésion, notamment des algues et/ou microorganismes, sur la surface. Il peut s'agir par exemple d'une surface préalablement traitée avec un produit chimique anti-adhésion (« antifouling »).

Le contenant peut être par exemple un contenant vertical ou horizontal ou incliné. Par exemple, l'inclinaison du contenant peut être comprise entre 0 et 90°. De préférence, le contenant est un contenant vertical ou horizontal. Il peut s'agir, par exemple, de tubes ou panneaux creux ou toute autre forme précitée, verticaux ou non, par exemple, l'inclinaison du contenant, par exemple des tubes ou autre, peut être comprise entre la vertical ou l'horizontal par exemple comprise entre 0 et 90°.

Par éclairage favorable à la culture d'algues et/ou de microorganismes, on entend l'éclairage naturel, par exemple la lumière du jour et/ou ou l'éclairage artificiel, par exemple émis par un moyen d'éclairage permettant de reproduire la lumière du jour ou une longueur d'onde suffisante pour la culture de l'algue et/ou du microorganisme.

Avantageusement, un moyen d'éclairage peut être un moyen indépendant des algues et/ou microorganismes en culture, venant en plus ou en remplacement de l'éclairage naturel par le soleil. Selon l'invention, le moyen d'éclairage peut être réalisé par exemple par un ou plusieurs tube(s) lumineux fluorescent(s), des diodes électroluminescentes (« LED »), par une ou plusieurs lampe(s) halogène(s). De préférence, l'éclairage peut être réalisé par un ou plusieurs tube(s) lumineux fluorescent(s), des diodes électroluminescentes, par une ou plusieurs lampe(s) halogène(s) dont les longueurs d'ondes lumineuses sont choisies entre 430 et 660 nm, de préférence égale à 430 nm ou 660 nm. L'éclairage peut être ornemental et/ou fonction de l'algue et/ou du microorganisme et de ses besoins pour sa croissance et / ou le traitement de l'effluent.

Le moyen d'éclairage peut être placée dans un espace créé par la disposition concentrique des contenants, par exemple des tubes et/ou du panneau creux. Il peut également être placé et/ou fixé sur une autre surface, par exemple une façade d'un bâtiment et/ou provenir du bâtiment lui-même. Par exemple lorsque l'on utilise deux contenant, par exemple tubes ou panneaux, disposés concentriquement, l'un étant externe et l'autre étant disposé à l'intérieur, le rétroéclairage peut être placé dans le contenant interne ou dans l'espace créé entre le contenant externe et le contenant interne, afin de protéger l'éclairage du milieu de culture.

Selon l'invention, les algues peuvent être des algues ou micro-algues ou un mélange de celle-ci.

Avantageusement, les algues peuvent être choisies par exemple dans le groupe comprenant des Chlorophycées, Chlorella, Parietochloris incisa, des Diatomées Amphora sp., Nitzchia sp., Chaetoceros sp., des Chrysophycées. En fait, avantageusement, selon l'invention, tout type d'algue convient, dès lors qu'elle peut être cultivée et traiter un effluent au sens de la présente invention. Avantageusement, il peut s'agir d'une ou d'un mélange de micro-algues permettant de former du biodiesel.

Avantageusement, le microorganisme peut être choisi parmi, par exemple, les bactéries, les levures, les champignons. Avantageusement, selon l'invention, tout type de microorganisme convient, dès lors qu'il peut être cultivée et traiter un effluent au sens de la présente invention. De préférence, le microorganisme est une bactérie. De préférence, la bactérie est une cyanobactérie. De préférence, la cyanobactérie est choisie dans le groupe comprenant *Spirulina platensis, Chroococcales Chamaesiphon, Chroococcales Gloeabacter, Chroococcales Synechococcus, Chroococcales Glocothece, Chroococcales Cyanothece, Chroococcales Gloecocapsa, Chroococcales Synechoexstis, Pleurocapsales Dermocarpa, Pleurocapsales Xenococccus, Pleurocapsales Dermocarpella, Pleurocapsales Myxosarcina, Pleurocapsales Chroococcidiopsis, Oscillatoriales Spirulina, Oscillatoriales Arthrospira, Oscillatoriales Oscillatoria, Oscillatoriales Lyngbya, Oscillatoriales Pseudanabaena, Oscillatoriales Starria, Oscillatoriales Crinalium, Oscillatoriales Microcoleus, Nostocales Anabaena, Nostocales Aphanizomenon, Nostocales Nodularia, Nostocales Cylindrospermum, Nostocales, Nostocales Scytonema, Nostocales Calothrix, Stigonematales Chlorogloecopsis, Stigonematales Fischerella, Stigonematales Stigonema, Stigonematales Geitleria, Plochloraceae Prochloron.*

La culture des algues et/ou des microorganismes peut être réalisée par tout moyen approprié connu de l'homme du métier. Avantageusement le milieu de culture peut être choisi en fonction de l'algue ou des algues pour permettre une culture de préférence optimale, mais surtout un métabolisme optimal pour le traitement de l'effluent, par exemple l'effluent gazeux. Les algues peuvent être stressées au cours de la culture pour augmenter leur efficacité de traitement de l'effluent gazeux ou liquide. Selon l'invention, le milieu de culture peut être choisi en fonction du microorganisme ou des microorganismes pour permettre une culture de préférence optimale, mais surtout un métabolisme optimal pour le traitement de l'effluent, par exemple l'effluent gazeux. Les microorganismes peuvent être stressés au cours de la culture pour augmenter leur efficacité de traitement de l'effluent gazeux ou liquide.

De très nombreux milieux de culture sont accessibles sur Internet et dans les ouvrages spécialisés. Selon l'invention, les cultures sont en milieu aqueux. Le stress peut être apporté par exemple au moyen de molécules chimiques. L'homme du métier connaît ces techniques et molécules.

Avantageusement, le moyen d'alimentation de la culture d'algues et/ou de microorganismes peut comprendre par exemple une pompe automatique, des moyens de régulation de l'alimentation des algues, un réservoir d'alimentation. Tout autre moyen approprié pour assurer la culture de l'algue convient. Tous ces moyens sont ceux classiquement utilisés par l'homme du métier pour assurer une culture continue d'algues ou de microorganismes.

Avantageusement, le moyen de régulation de la température de la culture d'algues et/ou de microorganismes peut être par exemple un thermostat ou tout autre moyen approprié de contrôle de la température et de réaction en cas de variation non désirée de la température.

Avantageusement, le module (D) photobioréacteur (PBR) d'isolation et/ou de régulation thermique d'un bâtiment de l'invention peut comprendre en outre un moyen de contrôle de la température autour du contenant de culture. Ces moyens de contrôle peuvent être connectés à des moyens de chauffage et/ou de refroidissement.

Avantageusement, le moyen de chauffage de la culture d'algues et/ou de microorganismes peut être choisi par exemple dans le groupe comprenant un moyen de récupération de la chaleur d'un bâtiment, un moyen de récupération de la chaleur extérieure, un moyen de récupération de la chaleur solaire, un moyen de récupération de l'énergie calorifique.

Par exemple, le moyen de récupération de la chaleur extérieure peut être une pompe à chaleur, par exemple, le moyen de récupération de la chaleur bâtiment peut être par exemple une double peau comprenant une enveloppe extérieure disposée devant une façade d'un bâtiment.

Avantageusement, le moyen de refroidissement de la culture d'algues et/ou de microorganismes peut être choisi par exemple dans le groupe comprenant un moyen de récupération de la fraicheur d'un bâtiment, un moyen de récupération de la fraicheur extérieure, un moyen de réfrigération, par exemple une climatisation. Par exemple, le moyen de récupération de la fraicheur extérieure peut être une pompe à chaleur.

Avantageusement, la présente invention permet de récupérer la chaleur et/ou la fraicheur d'un bâtiment pour cultiver les algues et/ou microorganisme et en même temps, grâce à la culture des algues et/ou microorganisme, de traiter des effluents générés, par exemple par ledit bâtiment.

Avantageusement, le module (D) photobioréacteur (PBR) d'isolation et/ou de régulation thermique (D) d'un bâtiment peut comprendre en outre un moyen de récupération de la biomasse formée par culture des algues.

Avantageusement, ledit module (D) photobioréacteur (PBR) d'isolation et/ou de régulation thermique (D) d'un bâtiment peut comprendre en outre un système de vidange du contenant de culture des algues et/ou microorganismes. Ce système peut permettre par exemple de nettoyer le contenant et/ou le module de la présente invention en entier. Il permet également, si souhaité, de récupérer la biomasse formée pour la réutiliser comme indiqué ci-dessus.

Ledit dispositif d'isolation et/ou de régulation thermique peut également comprendre un ou plusieurs des moyens de contrôle et régulation de la culture des algues suivants : moyens de contrôle de l'alimentation des algues et/ou microorganisme, moyens de contrôle de la température, moyens de contrôle du pH, moyens de contrôle de l'éclairage des algues et/ou microorganismes. Ces moyens peuvent être par exemple ceux couramment utilisés pour la culture d'algues et/ou de microorganismes, et de manière plus générale de microorganismes. Ledit dispositif d'isolation et/ou de régulation thermique peut également comprendre des moyens de contrôle de l'injection de l'effluent à traiter.

Avantageusement, ledit module (D) photobioréacteur (PBR) d'isolation et/ou de régulation thermique (D) d'un bâtiment peut être piloté par ordinateur afin de permettre une optimisation de la culture des algues et/ou microorganismes et/ou du traitement de l'effluent. L'ordinateur peut être connecté par exemple au thermostat du contenant de culture d'algues et/ou de microorganismes et aux différents moyens de contrôle installés pour faire fonctionner le dispositif de l'invention.

Avantageusement, le module (D) photobioreacteur (PBR) d'isolation et/ou de régulation thermique (D) d'un bâtiment de l'invention peut fonctionner en continu ou en discontinu. Il peut fonctionner, sans interruption, jour et nuit. L'éclairage des algues et/ou microorganismes peut ainsi être maintenu en permanence de manière à maintenir la culture active pour le traitement de l'effluent.

Avantageusement, la façade du bâtiment peut comprendre, avantageusement, un garde-corps. Le garde corps peut permettre, par exemple la circulation d'individus le long du dispositif, l'entretien et la maintenance du dispositif.

Par exemple, le garde corps peut, par exemple, être constitué de métal, de verre. Par exemple, il peut être constitué de menuiseries en métal et résille en acier, en métal, en inox, en galva, en nylon ou carbone, de barreaux en métal et/ou de menuiserie en métal et de membrane ETFE.

Avantageusement, le module (D) photobioréacteur (PBR) d'isolation et/ou de régulation thermique (D) d'un bâtiment peut comprendre en outre un renfort de structure permettant de supporter le contenant de culture d'algues et/ou de microorganismes, le contenant de culture d'algues et/ou de microorganismes pouvant être par exemple un tube de culture d'algues et/ou de microorganismes, ledit renfort pouvant être fixé au bâtiment. Ce renfort de structure peut être fixé sur le bâtiment, être autosupporté ou être fixé sur l'enveloppe extérieure de la double peau. Lorsque le contenant de culture des algues est disposé sur une façade d'immeuble, par exemple, cela permet de le supporter.

Le dispositif décrit peut comprendre en outre au moins une coursive, ladite coursive pouvant servir de moyen d'accès au contenant de culture d'algues et/ou de microorganismes pour du personnel d'entretien.

Ainsi, avantageusement, on peut former une véritable biofaçade d'un bâtiment, biofaçade qui permet non seulement de traiter l'air vicié émis dans et par le bâtiment, d'isoler le bâtiment, de récupérer la chaleur du bâtiment pour une culture d'algues et/ou de microorganismes, et permet en outre de fabriquer une biomasse réutilisable notamment en tant que biocarburant, dans le domaine de la pharmacie, dans le domaine agroalimentaire. Il peut d'agir par exemple de composés chimiques, de protéines.

Avantageusement la biomasse qui peut être formée par la culture d'algues et/ou de microorganismes peut en effet être récupérée et réutilisée, notamment comme biocarburant, comme composé chimique, composé pharmaceutique.

La biomasse peut permettre de fabriquer un microorganisme oléagineux.

Avantageusement, la biomasse peut être une biomasse transformable en charbon ou en biopétrole, par exemple par les techniques connues de l'homme du métier.

Avantageusement, la biomasse peut être utilisée directement pour produire de l'électricité. Elle peut également être acheminée, par exemple par un tuyau à un bâtiment, par exemple une centrale de traitement. Elle peut également être transformée, par exemple par pressage en huile ou traitement par pyrolyse par les techniques connues de l'homme du métier pour produire du charbon ou du biopétrole.

Selon l'invention, avantageusement, le dispositif peut être intégré à la structure d'un bâtiment ou rapporté sur le bâtiment.

La présente invention a également pour objet un bâtiment comprenant plusieurs modules (D) photobioréacteur (PBR) d'isolation et/ou de régulation thermique selon l'invention.

La présente invention permet ainsi de faire produire aux façades des bâtiments de l'énergie primaire biochimique obtenue par photosynthèse notamment de biocarburants.

Avantageusement, la présente invention permet d'utiliser les opportunités climatiques, chimiques et structurelles qu'offrent les façades des bâtiments, pour intégrer un procédé biochimique à l'intérieur ou le long des façades, de travailler sur un état de symbiose entre les deux systèmes qui se servent mutuellement, l'un recyclant les émanations de l'autre pour produire l'énergie dont il a besoin.

La présente invention peut avantageusement être mise en œuvre sur toutes les surfaces de bâtiments, enveloppes de bâtiments, façades ou toitures, ouvrage d'art souterrain, par exemple une infrastructure souterraine de circulation automobile, ferroviaire, par exemple un tunnel autoroutier, un tunnel de métro, neufs ou existant. Par exemple, la surface peut être une partie faiblement exploitée des bâtiments par exemple l'enveloppe extérieure ou toute autre surface ou toutes les surfaces du bâtiment ou une surface intérieur du bâtiment. La surface choisie pour placer le dispositif de l'invention est préférentiellement une surface qui permet de profiter d'une grande surface développée disponible, d'une disposition en hauteur, d'un rayonnement incident, d'émanations thermiques, de son infrastructure, et des apports chimiques du bâtiment. La surface peut être, par exemple, une surface en béton, une surface vitrée, une surface constituée par un complexe étanche et un bardage, une surface photovoltaïque. Les conditions optimales sont celles de la culture des algues choisies

En limitant l'emprise foncière au sol du dispositif de l'invention, cette invention peut trouver sa place n'importe où, même en milieu urbain déjà densifié. Elle peut en outre être appliquée à des façades d'immeubles existants lors d'opérations de réhabilitation ou restructurations. En cela elle répond aux campagnes de restructurations actuelles d'immeubles de bureaux pour les mettre aux nouvelles exigences et normes environnementales

Du point de vue du bâtiment, la présente invention peut avantageusement intégrer les évolutions récentes des constructions bioclimatiques. Par rapport aux façades énergétiques traditionnelles de type photovoltaïques, elle a l'avantage de pouvoir fonctionner sur n'importe quelle orientation.

Le contenant de culture lui-même peut être considéré comme un contenant d'eau et permet donc également de participer à la régulation des surchauffes thermiques.

Avantageusement, le dispositif de la présente invention peut comprendre en outre, par exemple, l'incorporation de moyens permettant une amélioration de la protection thermique des bioréacteurs.

D'autres avantages pourront apparaitre à la lecture des exemples ci-dessous.

### Brève description des figures :

- La figure 1 représente une façade Photo-Bio-Reacteur-Rideau ventilation alternée / élévation / coupe verticale / coupe horizontale.
- La figure 2 représente une façade Photo-Bio-Reacteur-Rideau ventilation alignée / élévation / coupe verticale / coupe horizontale.
- La figure 3 représente le détail d'une pièce moulée basse.
- La figure 4 représente des exemples de modules.
- La figure 5 représente des vues avec des modules en quinconces.
- La figure 6 représente des vues avec des modules en quinconces.
- La figure 7 représente un procédé mis en pratique sur la façade d'un immeuble de bureaux. En particulier, il s'agit d'une simulation des modules en quinconces sur un bâtiment de grande hauteur.
- La figure 8 représente une façade complète, en version double-façade, alternant Photo-Bio-Reacteurs-Rideau (D) avec des panneaux vitrés clairs ventilés (E) (élévation)
- La figure 9 représente une façade complète, en version double façade, constituée de panneaux vitrés multiparois clairs ventilés intégrant des Photo-Bio-Reacteurs-Rideau (D) amovibles (élévation)
- La figure 10 représente une façade comprenant les modules (D) et (E) en version double façade à mur-rideau, et à triple vitrage constituant le contenant de cultures d'algues / microorganismes, et à ventilation alignée (élévation / coupe verticale / coupe horizontale).
- La figure 11 représente une façade en version double façade à mur-rideau comprenant les modules (D) et (E) à vitrage multi-parois et PBR amovible à connectiques et échangeur thermique dorsal (élévation / coupe verticale / coupe horizontale).
- La figure 12 représente une façade comprenant les modules (D) et (E) en version simple façade à mur-rideau, et à vitrage multi-parois et PBR amovible à connectiques et échangeur thermique dorsal (élévation / coupe verticale / coupe horizontale).
- La figure 13 représente une façade en version simple façade sur châssis isolés, comprenant les modules (D) et (E) à connectiques et un module (X) échangeur thermique dorsal (élévation / coupe verticale / coupe horizontale).
- La figure 14 représente des exemples de formes géométriques de modules PBRR.
- La figure 15 représente neuf exemples de composition des parois du module (D).

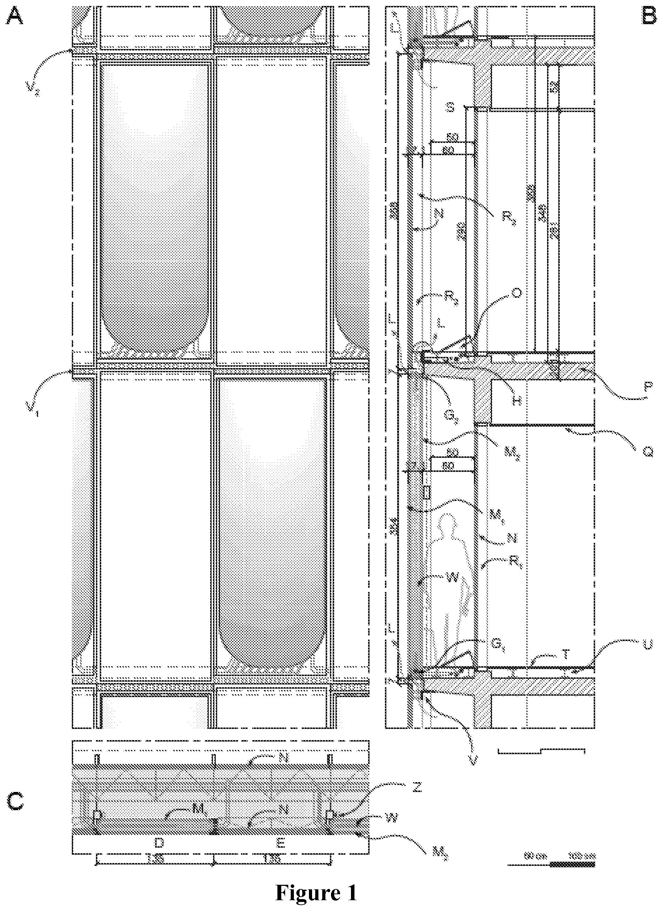

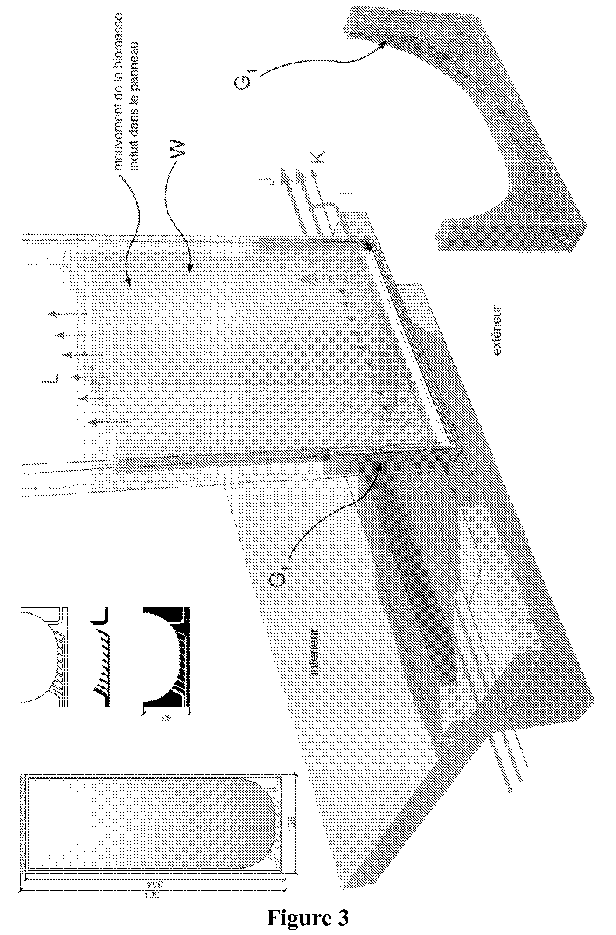

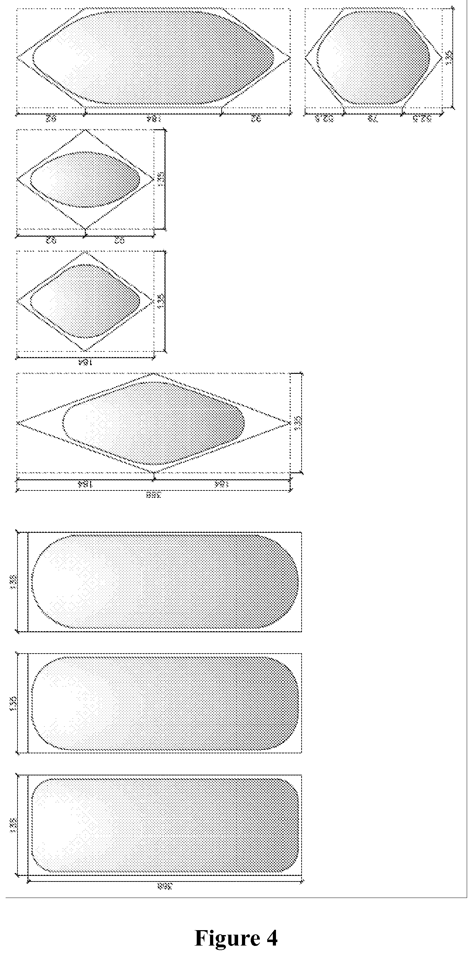

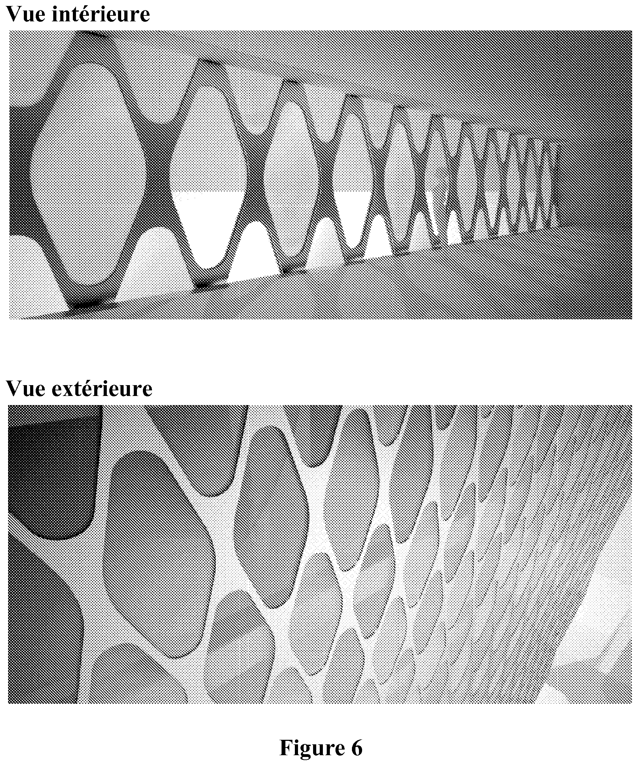

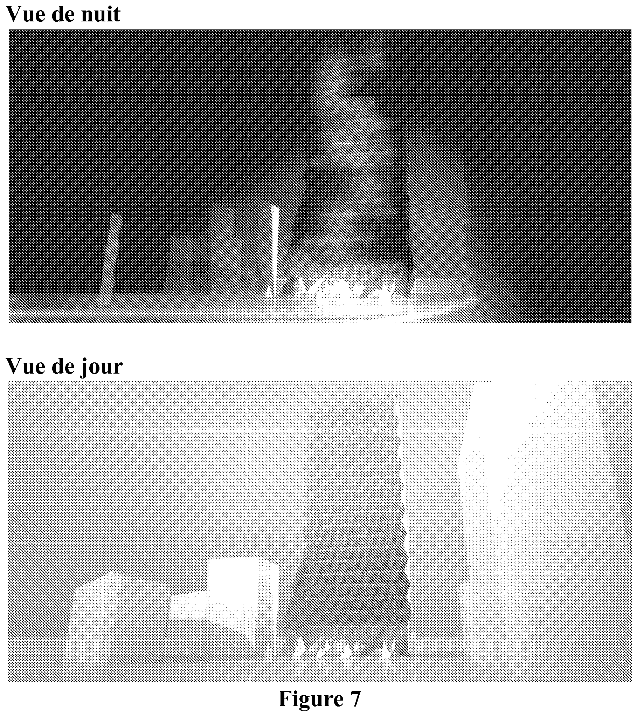

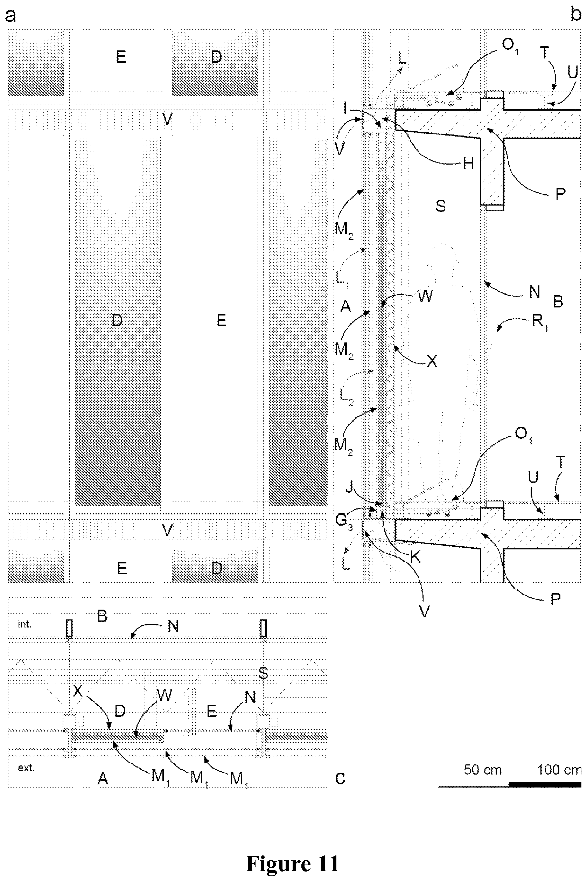

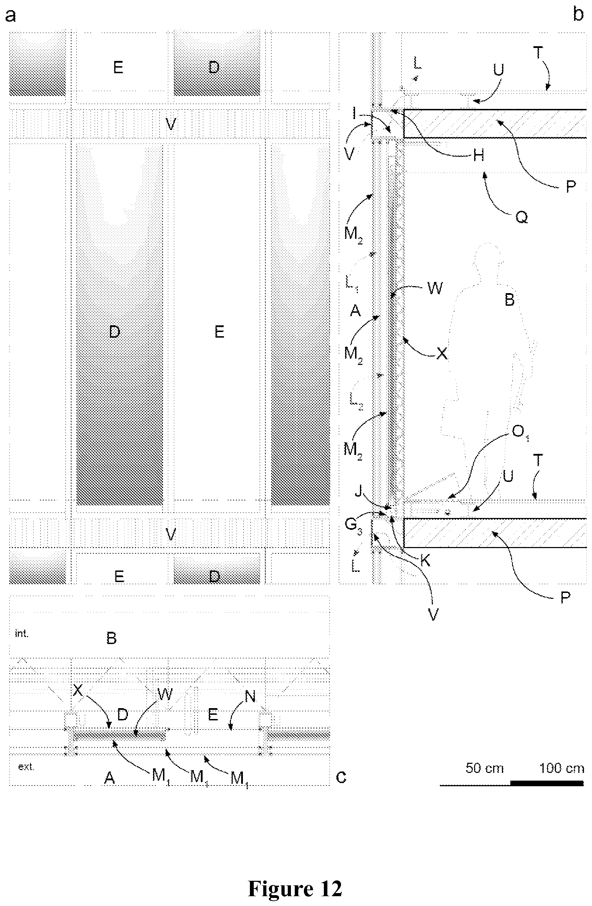

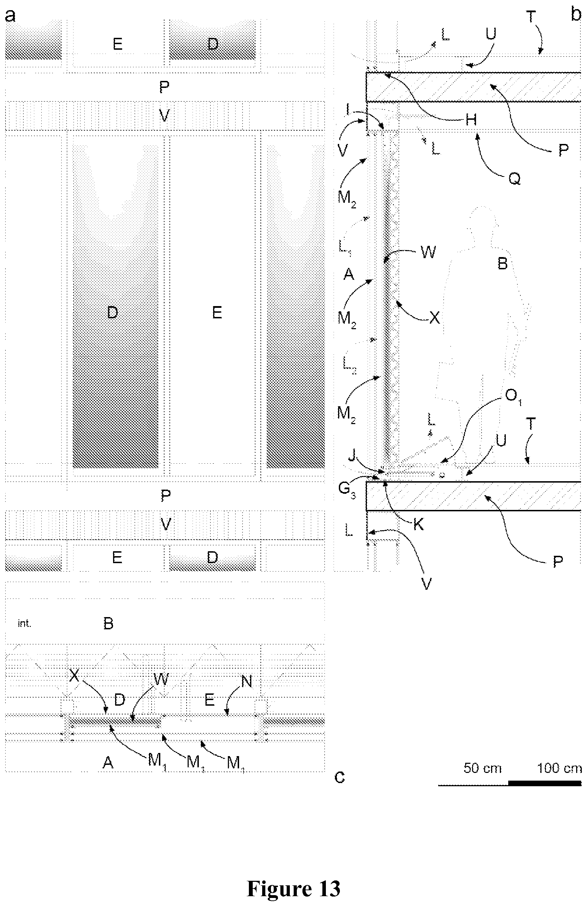

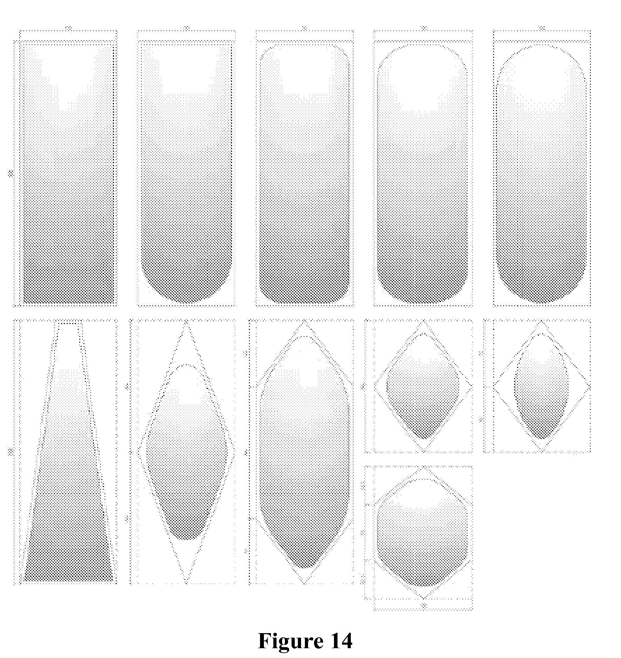

Les légendes ci-dessous s'appliquent à l'ensemble des figures 1 à 15 :
A façade extérieure d'une double-façade.
A' façade extérieure d'un bâtiment à simple façade.
B façade intérieure d'une double façade.
C ossature métallique ou en aluminium et à rupteur de pont thermique.
D module étanche façade photobioréacteurs également désigné module PBRR.
E module étanche façade ventilée également désigné module de mur-rideau étanche vitré ventilé.
F système d'accrochage des modules de mur-rideau (plaques, raidisseurs, aiguilles,...).
G1 pièce moulée téflon/résine/polycarbonate/befup (en pied du module).
G2 pièce moulée téflon/résine/polycarbonate/befup (en tête du module).
G3 pièce usinée en pied du module étanche façade photobioréacteurs _ métal/inox.
H sonde ou dispositif électronique de contrôle, capteurs thermiques, sondes...
I site d'injection (arrivée du milieu algal (eau + CO₂ dissout + nutriments)).
J site d'extraction du milieu algal.
K site injection d'air pour agitation du milieu dit par « air lift ».
L sortie d'air / ventilation naturelle.
L₁ lame d'air respirante.
L₂ lame d'air ventilée.
M1 double vitrage feuillete / double vitrage feuillete trempé dans un châssis étanche.
M2 simple vitrage feuilleté / simple vitrage feuilleté trempé dans un châssis étanche.
N simple vitrage / double vitrage dans un châssis étanche.
O trappe.
O1 trappe d'accès vide technique sous faux-plancher sur plots.
02 clapet/trappe d'entretien en tête du module de façade photo-bio-réacteur (module D).
P dalle béton.
Q faux-plafond.
R1 montant acier/aluminium.
R2 montant acier/aluminium.
S double peau / chemin technique.
T faux plancher.
U plots.
V ventilation naturelle/ profilé à ventelles.
W micro algues.
X double capot métallique intégrant un circuit de fluide caloporteur pour échange thermique avec le circuit d'eau du bâtiment.
Z rail ou rail avec sonde à ultrasons ou 3 tubes fluo UV disposés toute hauteur le long du module de façade photo-bio-réacteur / dispositif de nettoyage «anti-fooling».
   (a) algues.
   (b) double vitrage.
   (c) simple vitrage.
   (d) bac étanche en polycarbonate soudé.
   (e) plaque métallique.
   (f) capot inox embouti ou inox plié anti-feu.
   (g) lame d'air ventilée.
   (h) lame d'air respirante.
   (i) panneau polycarbonate.
   (j) bac en composite transparent embouti ou ressoudé fin.
   (k) vitrage Quantum ou toile de fibre optique intégré dans un panneau Acrylique ou plaque inox.
   (l) circuit fluide caloporteur pour échange thermique.

### EXEMPLE

### Exemple 1: Dispositif en mur rideau vertical à trame verticale alternée

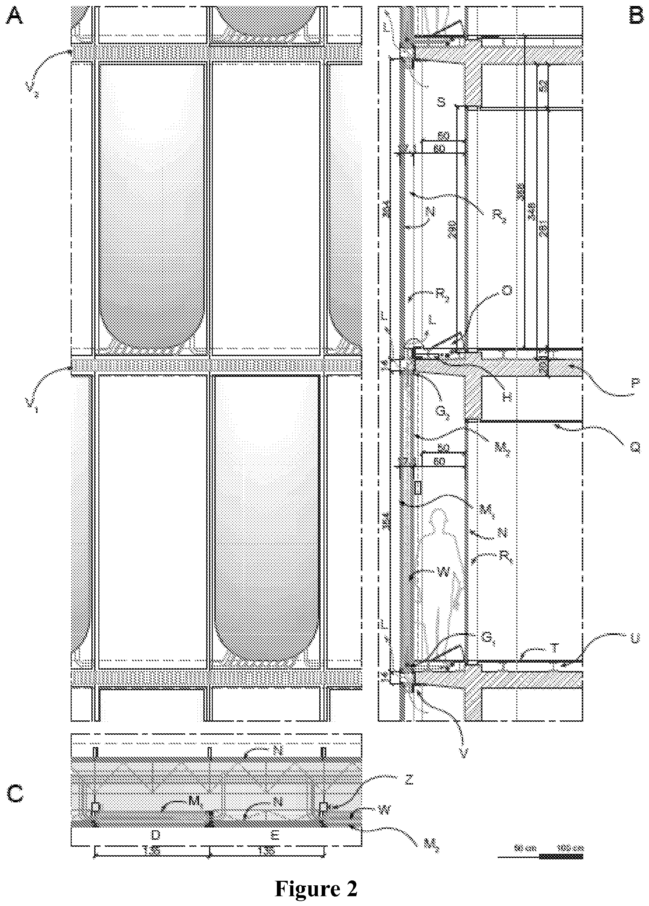

On décrit ici des modes de réalisation du module photobioréacteur (PBR) d'isolation et/ou de régulation thermique. Le dispositif utilisé est schématisé sur les figures 2, 8 et 10 et constitue la façade d'un bâtiment.

La culture d'algue utilisée est Chlorella. Elle est cultivée dans un milieu aqueux de culture, à savoir le milieu de Walnes c'est-à-dire pour 10 litres de milieu 680g de nitrate de sodium (NaNO3), 200g de Di-hydrogénophosphate de sodium, 400g d'acide Ethylènediamine tetraacetique de sodium (Na₂EDTA), 20g d'acide borique (H3BO3), 40 ml d'une solution de 500 ml contenant 32,5 g de Bromure de potassium (KBr), 6,5g de chlorure de strontium (SrCl₂, 6H20), 0,25g de solution de chlorure d'aluminium (AICI3, 6 H20), 0,1g de chlorure de rubidium (RbCI), 0,05 g de chlorure de lithium (LiCI, H20), 0,025g de iodure de potassium (KI) et 800 mL d'une solution de 10 litres contenant 213,2 g de chlorure de fer hexahydraté (FeCl₃, 6 H₂0), 15,0 g de sulfate de manganèse monohydraté (MnSO₄, H₂O), 2,5 g de sulfate de zinc (ZnSO4), 2,0 g de sulfate de cuivre pentahydraté (CuSO₄, 5H20), 0,26 g de sulfate de cobalt heptahydraté (CoSO₄, 7 H₂0), 0,14 g de molybdate de sodium dihydraté (Na₂Mo₄, 2H₂0) et 0,10g de fluorure de sodium (NaF).

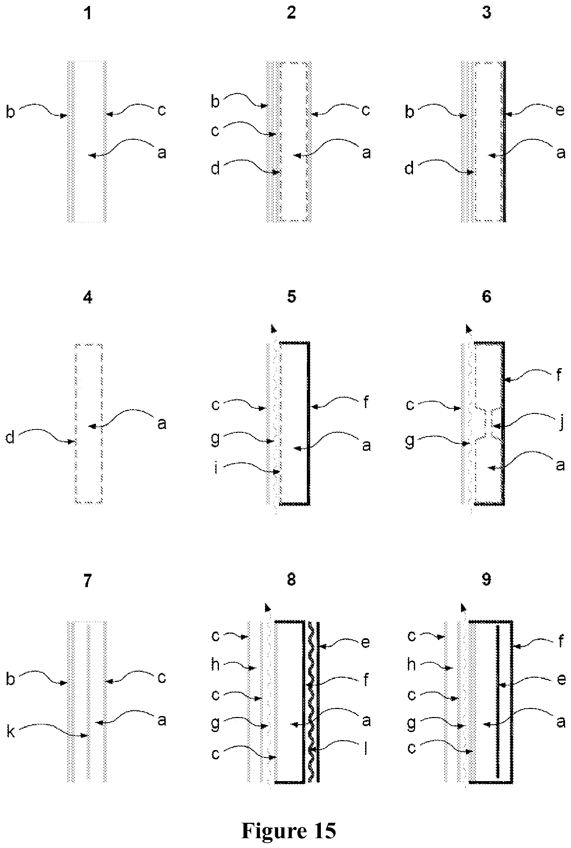

La culture de l'algue est effectuée dans le milieu précédent à une température allant de 18 à 25°C, de préférence 20°C. Le pH du milieu est égal à 7,3.
Un module (D) décrit est un module préfabriqué (figure 15-1), constitué de deux parois (M1), côté extérieur, et (M2), côté bâtiment, parclosées respectivement par l'extérieur et par l'intérieur au sein d'un cadre en profilé aluminium. La paroi (M1) est constituée d'un double vitrage étanche associant deux verres de type « extra clairs diamant » de Saint Gobain Glass, l'un, côté extérieur, de 4 mm et l'autre, côté milieu de culture, de 23 mm d'épaisseur, les deux vitrages étant séparés d'une lame d'air étanche remplie d'air de 12 mm. La paroi (M2), côté bâtiment, est constituée d'un vitrage feuilleté d'une épaisseur de 23 mm. L'espace entre les deux parois est rendu étanche par des joints de silicone et permet d'accueillir le milieu de culture comprenant les algues (W). Ce mode de réalisation est adapté aux pays tempérés.

Dans un autre mode de réalisation, le module (D) est un module préfabriqué constitué d'une paroi (M1), côté extérieur, et d'une seconde paroi (M2), côté bâtiment, parclosées respectivement par l'extérieur et par l'intérieur au sein d'un cadre en profilé aluminium (figure 15-2). La paroi (M1) est constituée d'un triple vitrage feuilleté associant trois verres de type « extra clairs diamant » de marque Saint Gobain Glass, les deux premiers côté extérieur de 4 mm d'épaisseur, et le troisième côté milieu de culture de 10 mm d'épaisseur, les trois vitrages étant séparés par deux lames d'air étanche remplies d'air de 12 mm. La paroi (M2), côté bâtiment, est constituée d'un simple vitrage feuilleté d'une épaisseur de 10 mm. L'espace entre les deux parois comprend un contenant de culture en polycarbonate, comprenant le milieu de culture comprenant les algues (W). Le contenant de culture en polycarbonate assure l'étanchéité. Dans ce mode de réalisation, l'étanchéité en conséquence ne doit pas être renforcée au niveau des vitrages eux-mêmes comme dans le mode de réalisation précédent. Ce mode de réalisation est particulièrement adapté aux pays froids.

Dans un autre mode de réalisation, le module (D) est un module préfabriqué constitué d'une paroi (M1), côté extérieur, et d'une seconde paroi (M2), côté bâtiment, parclosées respectivement par l'extérieur et par l'intérieur au sein d'un cadre en profilé aluminium (figure 15-3). La paroi (M1) est constituée d'un triple vitrage feuilleté associant trois verres de type « extra clairs diamant » de marque Saint Gobain Glass, les deux premiers côté extérieur de 4 mm d'épaisseur, et le troisième côté milieu de culture de 10 mm d'épaisseur, les trois vitrages étant séparés par deux lames d'air étanche remplies d'air de 12 mm. La paroi (M2), côté bâtiment, est constituée d'une plaque d'acier de 4 mm. L'espace entre les deux parois comprend un contenant de culture en polycarbonate, comprenant le milieu de culture, comprenant les algues (W). Le contenant de culture en polycarbonate assure l'étanchéité. Dans ce mode de réalisation, l'étanchéité en conséquence ne doit pas être renforcée au niveau des vitrages eux-mêmes comme dans le premier mode de réalisation. Ce mode de réalisation est particulièrement adapté aux pays froids.

Dans un autre mode de réalisation, le module (D) est un module préfabriqué « multi-parois » constitué d'une paroi (M1), côté extérieur, et d'un contenant de culture faisant office de seconde paroi (M2), côté bâtiment, parclosés respectivement par l'extérieur et par l'intérieur au sein d'un cadre en profilé aluminium (figure 15-5). La paroi (M1) est constituée d'un simple vitrage feuilleté de 4 mm d'épaisseur de type « extra clairs diamant » de marque Saint Gobain Glass. La paroi (M2), côté bâtiment, est le contenant de culture comprenant les algues (W), constitué d'une plaque d'acier de 4 mm pliée, resoudée, définissant ainsi un capot métallique, et disposant en face avant d'une paroi de polycarbonate fixée sur ledit capot de manière étanche. L'espace entre les deux parois est une lame d'air ventilée. Ce mode de réalisation est particulièrement adapté aux pays chauds.

Dans un autre mode de réalisation, Le module (D) est un module préfabriqué (figure 15-7), constitué de deux parois (M1), côté extérieur, et (M2), côté bâtiment, parclosées respectivement par l'extérieur et par l'intérieur au sein d'un cadre en profilé aluminium. La paroi (M1) est constituée d'un double vitrage étanche associant deux verres de type « extra clairs diamant » de Saint Gobain Glass, l'un côté extérieur de 4 mm et l'autre côté milieu de culture de 23 mm d'épaisseur, les deux vitrages étant séparés d'une lame d'air étanche remplie d'air de 12 mm. La paroi (M2), côté bâtiment, est constituée d'un vitrage feuilleté d'une épaisseur de 23 mm. L'espace entre les deux parois est rendu étanche par des joints de silicone et permet d'accueillir le milieu de culture comprenant les algues (W). Cet espace comprend également, parallèlement et au centre de l'espace, un vitrage Quantum Glass de Saint Gobain Glass, ou réseau de fibre optique intégré dans un panneau acrylique. Cet éclairage au sein du milieu de culture permet de prolonger l'exposition des algues au rayonnement lumineux dans le spectre de la lumière visible dès que le rayonnement lumineux naturel diminue (journée nuageuse ou pluvieuse, soirées, voire nuits), afin de maintenir une productivité plus stable d'algues. Ce mode de réalisation est adapté aux pays tempérés et froids, à plus faible ensoleillement.

Dans un autre mode de réalisation, Le module (D) est un module préfabriqué (figure 15-8), constitué de deux parois (M1), côté extérieur, et (M2), côté bâtiment, parclosées respectivement par l'extérieur et par l'intérieur au sein d'un cadre en profilé aluminium. La paroi (M1) est constituée d'un vitrage multi-parois associant deux verres de type « extra clairs diamant » de Saint Gobain Glass de 4 mm d'épaisseur, les deux vitrages étant séparés par une lame d'air respirante de 40 mm d'épaisseur, c'est-à-dire remplie d'air à hygrométrie similaire à l'air extérieur, afin d'éviter la formation de condensation. La paroi (M2), côté bâtiment, est le contenant de culture comprenant les algues (W), constitué d'une plaque d'acier de 6 mm pliée, resoudée, définissant ainsi un capot métallique, et disposant en face avant d'un vitrage feuilleté de 2x10 mm d'épaisseur séparé par un feuille de polyvinyle de butyral (PVB) de 0,38 mm d'épaisseur de marque Evasafe du fabricant Centriglass. L'espace entre les deux parois est une lame d'air ventilée. Un serpentin soudé en face arrière de ce capot métallique assure la circulation d'un fluide caloporteur antigel, anti corrosion, bactéricide, fongicide, anti tartre, à base de mono propylène glycol, de marque MB444D du fabricant PCMB. Ce fluide caloporteur permet d'assurer un échange thermique entre le milieu de culture des algues et différents réseaux de fluides du bâtiment (circuit d'eau froide, circuit d'eau chaude sanitaire).

Dans un autre mode de réalisation, Le module (D) est un module préfabriqué (figure 15-9), constitué de deux parois (M1), côté extérieur, et (M2), côté bâtiment, parclosées respectivement par l'extérieur et par l'intérieur au sein d'un cadre en profilé aluminium. La paroi (M1) est constituée d'un vitrage multi-parois associant deux verres de type « extra clairs diamant » de Saint Gobain Glass de 4 mm d'épaisseur, les deux vitrages étant séparés par une lame d'air respirante de 40 mm d'épaisseur, c'est-à-dire remplie d'air à hygrométrie similaire à l'air extérieur, afin d'éviter la formation de condensation. La paroi (M2), côté bâtiment, est le contenant de culture comprenant les algues (W), constitué d'une plaque d'acier de 4 mm pliée, resoudée, définissant ainsi un capot métallique, et disposant en face avant d'un double vitrage de 2x10 mm d'épaisseur séparé par une lame d'air étanche de 12 mm d'épaisseur de marque Saint Gobain Glass. L'espace entre les deux parois est une lame d'air ventilée. Le milieu de culture est contenu entre ce double vitrage et une plaque métallique soudée de 3 mm d'épaisseur à l'intérieur du capot, qui permet de rigidifier le capot.

Dans le premier mode de réalisation, l'espace situé entre les parois M1 et M2 incorpore également à sa base une pièce moulée en polycarbonate (G1) assurant la synthèse des circuits d'injection de l'air servant à l'agitation du milieu selon la technique dite de « air lift », ainsi que les sorties de vidange et de collecte du milieu de culture (W). Les tuyaux de fluides sont formés directement en creux pendant le moulage de la pièce (G1).

Le circuit d'injection d'air est de type arborescent, à partir du point d'injection situé en face arrière du module, et ainsi divise le flux principal d'air en huit flux jusqu'au nu de la surface horizontale supérieure de la pièce moulée. L'angle d'inclinaison de ces injecteurs est incliné de 30 degrés par rapport à la verticale en élévation, afin de créer un mouvement laminaire du milieu de culture, et incliné de 30 degrés par rapport à la verticale en coupe verticale, afin de diriger le flux d'air vers la vitre intérieure de la paroi M1, et éviter la formation de dépôts d'algues ou microorganismes sur cette vitre. La section des trous d'injection est de 1,5 mm.

Un trou de vidange/récolte du milieu de culture de 20 mm de section est situé à l'extrémité gauche de la pièce moulée en polycarbonate (G1). Il permet d'assurer la vidange / récolte du milieu par gravité ou pompage.

Un trou d'injection de CO₂ dissous de 5 mm de section est situé à l'extrémité droite de la pièce moulée. Il permet d'injecter du CO₂ afin de modifier de manière contrôlée le pH du milieu de culture.

Cette pièce moulée permet d'assurer une connectique optimisée avec les circuits de fluides placés sous le faux-plancher technique (T) de la coursive (S), et accessibles grâce à une trappe d'accès (O1).

Cet espace incorpore également à sa tête une pièce moulée en polycarbonate (G2) assurant la synthèse des circuits d'injection d'eau et de milieu, ainsi que de collecte du trop plein du milieu et des gaz émis par le milieu de culture, et permettant enfin la fixation d'une sonde pH et température plongeant dans le milieu. Les tuyaux de fluides sont formés directement en creux pendant le moulage de la pièce (G2).

L'injection d'eau s'effectue par un tube vertical d'une section de 20 mm formé en creux dans la pièce moulée (G2). L'injection de milieu s'effectue par un tube vertical d'une section de 6 mm formé en creux dans la pièce moulée (G2). Un trou de 20 mm de section permet d'évacuer le trop plein de milieu en cas de défaillance du contrôle de niveau d'eau. Un trou fileté de 10 mm permet de récupérer les gaz générés par les algues ou microorganismes. Un trou fileté traversant vertical de 10 mm permet de venir visser une sonde à pH et température, plongeant par le haut dans le milieu de culture, et permettant de contrôler en continu la température et le pH du milieu de culture.

Cette pièce moulée permet d'assurer une connectique optimisée avec les circuits de fluides placés sous le faux-plancher technique (T) de la coursive (S) de l'étage supérieur, et accessibles au sommet du module, grâce à une trappe d'accès (02), et via l'étage supérieur, par la trappe d'accès (O1).

Des sondes sont connectées à un dispositif de contrôle permettant de suivre l'évolution du pH, de la concentration du CO₂ et de la température du milieu.

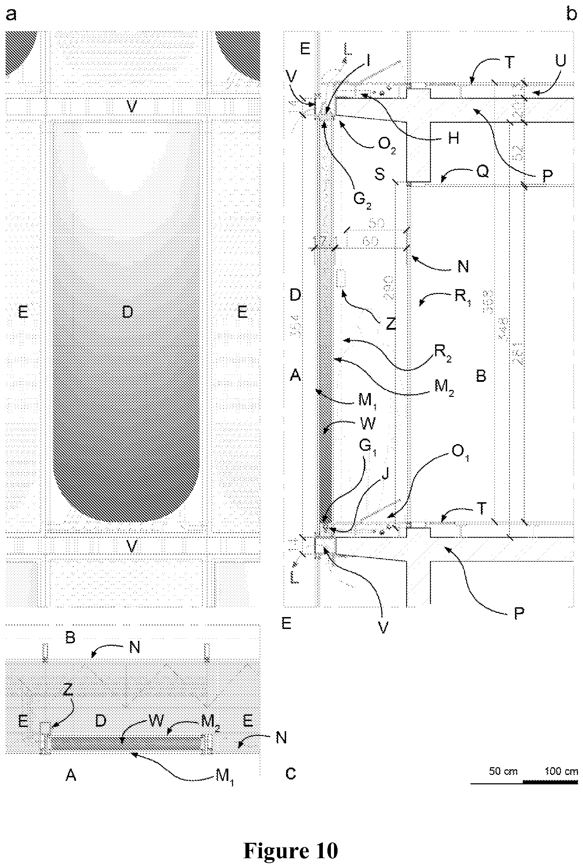

Le module (E) est un module préfabriqué comprenant une paroi (N) parclosée par l'intérieure au sein d'un cadre en profilé aluminium. La paroi N est constituée d'un double vitrage étanche, représenté sur la figure 2 et 10, associant deux verres de 4 mm d'épaisseur à couche basse émissivité de Saint Gobain Glass, les deux vitrages étant séparés d'une lame étanche remplie d'argon de 16 mm.

Le module (E) est constitué dans sa partie supérieure et inférieure de cavités équipées de clapets à ventelles (V) dont l'ouverture peut être contrôlée mécaniquement par un système de gestion technique du bâtiment de marque Vista/Xenta du fabricant Schneider Electric .afin d'assurer la régulation thermique passive de l'espace en coursive, soit en les ouvrant l'été pour extraire l'air chaud par convexion naturelle (L), soit en les fermant l'hiver pour bénéficier de l'effet de serre au sein de la double-peau (S).

Les modules (D) et (E) préfabriqués sont des panneaux de mur-rideau venant se fixer de manière standard en nez de dalles par un système de plaques métalliques et accroches, ainsi qu'entre eux.

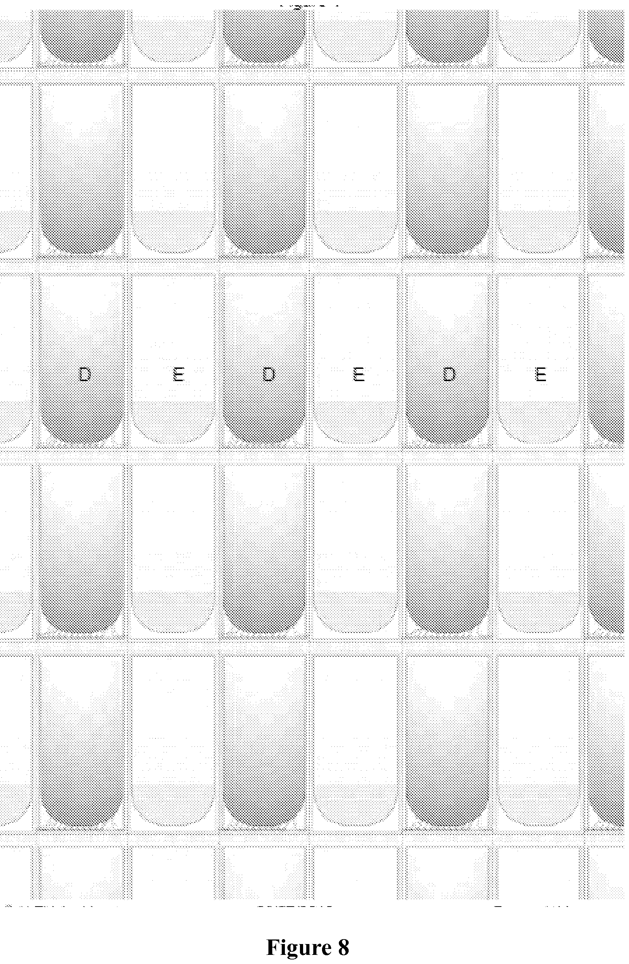

Les modules (E) et (D) constituent la façade extérieure du bâtiment via une disposition horizontale et alterné de chacun des modules (figure 8).

Le bâtiment comprend également un simple vitrage intérieur (N) monté sur châssis aluminium, situé entre chaque dalle de niveau.

L'espace situé entre les modules (E) et (D) et les vitrages (N) comprend une coursive permettant le passage technique pour l'entretien et également définissant un espace tampon dans lequel est régulée la température de manière passive par l'ouverture contrôlée des ventelles (V).

La coursive est une extension de la dalle en béton fabriqué qui est fixée dans l'ossature primaire.

Les cuves de milieu de culture concentré sont situées dans un local technique ventilé en toiture. Le milieu de culture est préparé afin d'avoir toutes les caractéristiques adéquates du milieu de culture de production décrit plus haut, une fois dilué dans les contenants des milieux de culture au sein des modules (D). En particulier, le milieu de culture concentré dispose d'algues ou microorganismes, de nutriments, et de CO₂ dissout.

Une pompe d'alimentation « pneumatique alimentaire » du fabricant TechniFlow approvisionne les contenants de milieu de culture des modules (D) via un réseau hydraulique contrôlé, permettant de remplir les contenants des modules (D) individuellement, étage par étage.

L'injection d'eau dans les contenants de milieu de culture des modules (D) s'effectue grâce à une connexion sur le réseau d'eau du bâtiment, étage par étage, à partir de l'étage supérieur à celui où est disposé chaque module (D). L'injection d'eau se fait via le réseau d'eau, sous pression normale, du bâtiment de manière contrôlée et simultanée à celle du milieu de culture, afin que le milieu de culture de production ait toutes les caractéristiques adéquates décrit plus haut, une fois dilué dans les contenants des milieux de culture au sein des modules (D).

La collecte du milieu de culture s'effectue de manière contrôlée via une pompe « pneumatique alimentaire » du fabricant TechniFlow située à l'extrémité de chaque coursive. Des vannes à boisseau sphérique 2 voies en plastique à commande électrique (électrovannes) de marque TORK permettent d'effectuer une collecte / vidange individuelle de chaque contenant de milieu de culture de module (D), étage par étage. Le milieu de culture est acheminé via le réseau hydraulique dédié jusqu'à une cuve située dans un local technique en sous-sol.

Un extracteur d'air de référence HF.EA.11570 du fabricant Asecos GmbH est disposé sur le conduit d'évacuation des effluents gazeux de chaque étage du bâtiment. Il s'agit d'un extracteur permettant l'acheminement de l'effluent dans une gaine de section adaptée à la taille des locaux jusqu'à un compresseur d'air à piston lubrifié (stationnaire) 125 l/min, 10 - 15 bar | SRD 125 series (ou supérieur selon la longueur de la coursive) du fabricant BOGE compressed air systems situé à l'extrémité de chaque coursive. Ce compresseur permet d'injecter de manière contrôlée cet effluent gazeux dans chaque contenant de culture via la pièce moulée (G1), permettant ainsi d'augmenter et ou diminuer le débit de l'effluent en fonction de la concentration du CO2 dans le milieu de culture, mesurée via la sonde pH. Ainsi, on récupère le CO₂ du bâtiment pour l'injecter dans le dispositif de l'invention.

Les gaz générés par les cultures de microalgues sont extraits via surpression naturelle en surface du milieu de culture par l'orifice prévu à cet effet au sein de la pièce (G2). Dans le cas de la Chlorelle, ce gaz est fortement chargé en oxygène.

La culture des algues est effectuée dans le milieu précité et la biomasse récupérée par pompage via une pompe « pneumatique alimentaire » du fabricant TechniFlow du milieu dans le réservoir de stockage situé dans le local technique en sous-sol.

Enfin, un rail (Z) mobile horizontalement sur toute la longueur de la coursive est équipé d'une buse de pulvérisation à ultrasons de marque Sinaptec Ultrasonic technology, piloté par un générateur électronique de la gamme NexTgen du même fabricant, lui-même mobile sur ce rail verticalement, permettant d'assurer si besoin le nettoyage des contenants de culture en éliminant les microalgues qui se seraient collées aux verres des parois (M1) ou (M2).

De manière surprenante, les inventeurs ont montré que la disposition des modules en façade du bâtiment permet d'augmenter son rendement énergétique. En effet, la double-peau ainsi créée assure à la fois une régulation thermique passive du bâtiment par effet de serre l'hiver et ventilation naturelle l'été, ainsi qu'une régulation thermique active du bâtiment par la présence d'eau maintenue entre 18 et 25°C dans le murrideau.

L'apport de CO₂ et de NO₂ par l'effluent gazeux dans le milieu de culture contenant les algues en présence de soleil et/ou sous éclairage avec les diodes, permet de recycler le CO₂ et le NO₂ tout en produisant de la matière organique émettant de l'oxygène par les algues. Ce recyclage est fait naturellement par les algues via le procédé de photosynthèse. Cet effluent permet en outre de chauffer le milieu de culture des algues.

Cet exemple démontre donc clairement que le module selon l'invention permet à la fois d'isoler et réguler thermiquement le bâtiment mais également de recycler les effluents gazeux issus de bâtiments traités par le module de la présente invention.

## Revendications

1. Module (D) photobioréacteur (PBR) d'isolation et/ou de régulation thermique d'un bâtiment, ledit module (D) comprenant un contenant de culture d'algues et/ou de microorganismes (W) en milieu aqueux, au moins deux parois parallèles ou sensiblement parallèles définissant au moins un espace intérieur, ledit au moins un espace intérieur comprenant ledit contenant de culture d'algues et/ou de microorganismes (W) en milieu aqueux, l'une de ces parois pouvant servir de paroi de façade ou être apposée à une paroi de façade du bâtiment, lesdites au moins deux parois étant des parois multicouches, **caractérisé en ce que** lesdites au moins deux parois multicouches définissant une ou plusieurs lames d'air (L₂, L₁).

2. Module (D) selon la revendication 1 dans lequel le contenant de culture d'algue et/ou de microorganismes se présente sous une forme choisie dans le groupe comprenant un tube plat, un panneau creux, un parallélépipède rectangle, un parallélépipède rectangle avec bord arrondis et une forme creuse sans arrête vive.

3. Module (D) selon la revendication 1 ou 2 dans lequel l'épaisseur des parois sont indépendamment de 0,5 mm à 50 mm.

4. Module (D) selon l'une quelconque des revendications précédentes, dans lequel le bâtiment est un bâtiment industriel et/ou de bureaux et/ou d'habitation et/ou agricole et/ou un ouvrage d'art et/ou mixte.

5. Module (D) selon l'une quelconque des revendications précédentes, dans lequel une pièce moulée (G1, G2, G3) est incorporée dans le contenant de culture d'algues et/ou de microorganismes.

6. Module (D) selon la revendication 5, dans lequel la pièce moulée (G1, G2, G3) comprend des tuyaux permettant d'alimenter le contenant de culture d'algues et/ou de microorganismes.

7. Module (D) selon l'une quelconque des revendications précédentes, dans lequel les algues (W) sont choisies dans le groupe comprenant des Chlorophycées, Chlorella, Parietochloris incisa, des Diatomées Amphora sp., Nitzchia sp., Chaetoceros sp., des Chrysophycées.

8. Module (D) selon l'une quelconque des revendications précédentes, dans lequel chaque paroi comprend indépendamment un simple (M₁), double (M₂) ou triple (M₃) vitrage, un vitrage multi-parois, ou encore un assemblage de ces différents vitrages, lesdites parois multicouches définissant une ou plusieurs lames d'air

9. Utilisation d'un module (D) photobioréacteur (PBR) d'isolation et/ou de régulation thermique selon l'une quelconque des revendications 1 à 8 pour la fabrication d'un bâtiment.

10. Façade de bâtiment comprenant un module (D) photobioréacteur (PBR) d'isolation et/ou de régulation thermique selon l'une quelconque des revendications 1 à 8.

11. Bâtiment comprenant plusieurs modules (D) photobioréacteur (PBR) d'isolation et/ou de régulation thermique tel que défini selon l'une quelconque des revendications 1 à 8.

## Patentansprüche

1. Photobioreaktor (PBR)-Modul (D) zur Isolierung und/oder Regulierung der Temperatur eines Gebäudes, wobei das Modul (D) einen Behälter zum Züchten von Algen und/oder Mikroorganismen (W) in einem wässrigen Medium umfasst, wobei mindestens zwei parallele oder im Wesentlichen parallele Wände mindestens einen Innenraum definieren wobei der mindestens eine Innenraum den Behälter zur Kultivierung von Algen und/oder Mikroorganismen (W) in einem wässrigen Medium umfasst, wobei eine dieser Wände als Fassadenwand dienen kann oder an einer Fassadenwand des Gebäudes befestigt werden kann, wobei die mindestens zwei Wände mehrschichtige Wände sind, **dadurch gekennzeichnet dass** die mindestens zwei mehrschichtigen Wände einen oder mehrere Luftspalte (L2, L1) definieren.

2. Modul (D) nach Anspruch 1, wobei der Algen- und/oder Mikroorganismen-Kulturbehälter eine Form aufweist, die aus der Gruppe ausgewählt ist, die aus einem flachen Rohr, einer hohlen Platte, einem rechteckigen Parallelepiped, einem rechteckigen Parallelepiped mit abgerundeten Kanten und einer hohlen Form ohne scharfe Kanten besteht.

3. Das Modul (D) nach Anspruch 1 oder 2, wobei die Dicke der Wände unabhängig voneinander 0,5 mm bis 50 mm beträgt.

4. Das Modul (D) nach einem der vorhergehenden Ansprüche, wobei das Gebäude ein Industrie- und/oder Büro- und/oder Wohn- und/oder Landwirtschafts- und/oder Technikgebäude und/oder ein Gebäude mit gemischter Nutzung ist.

5. Modul (D) nach einem der vorhergehenden Ansprüche, wobei ein Formteil (G1, G2, G3) in den Algen- und/oder Mikroorganismen-Kulturbehälter eingebaut ist.

6. Modul (D) nach Anspruch 5, wobei das Formteil (G1, G2, G3) Rohre zur Versorgung des Algen- und/oder Mikroorganismenkulturbehälters aufweist.

7. Modul (D) nach einem der vorhergehenden Ansprüche, wobei die Algen (W) aus der Gruppe ausgewählt sind, die Chlorophyceae, Chlorella, Parietochloris incisa, Diatomeen Amphora sp., Nitzchia sp., Chaetoceros sp., Chrysophyceae umfasst.

8. Modul (D) nach einem der vorhergehenden Ansprüche, bei dem jede Wand unabhängig eine einfache (M1), doppelte (M2) oder dreifache (M3) Glasscheibe, eine mehrwandige Glasscheibe oder eine Anordnung dieser verschiedenen Glasscheiben umfasst, wobei die mehrschichtigen Wände eine oder mehrere Luftlamellen definieren

9. Verwendung eines Photobioreaktor (PBR)-Moduls (D) zur Isolierung und/oder Wärmeregulierung nach einem der Ansprüche 1 bis 8 zur Herstellung eines Gebäudes.

10. Gebäudefassade mit einem Photobioreaktor (PBR)-Modul (D) zur Isolierung und/oder Wärmeregulierung nach einem der Ansprüche 1 bis 8.

11. Gebäude mit einer Mehrzahl von isolierenden und/oder thermisch regulierenden Photobioreaktor (PBR)-Modulen (D) nach einem der Ansprüche 1 bis 8.

## Claims

1. Photobioreactor (PBR) module (D) for insulating and/or regulating the temperature of a building, said module (D) comprising an algal and/or microorganism culture container (W) in an aqueous medium, at least two parallel or substantially parallel walls defining at least one interior space said at least one interior space comprising said algal and/or microorganism culture container (W) in an aqueous medium, one of these walls being able to serve as a facade wall or to be affixed to a facade wall of the building, said at least two walls being multi-layer walls, **characterized in that** said at least two multi-layer walls defining one or more air space (L₂, L₁).

2. The module (D) according to claim 1 wherein the algal and/or microorganism culture container is in a form selected from the group comprising of a flat tube, a hollow panel, a rectangular parallelepiped, a rectangular parallelepiped with rounded edges and a hollow shape without sharp edges.

3. A module (D) according to claim 1 or 2 wherein the thickness of the walls is independently from 0.5 mm to 50 mm.

4. Module (D) according to any one of the preceding claims, wherein the building is an industrial and/or office and/or residential and/or agricultural and/or engineering and/or mixed-use building.

5. A module (D) according to any one of the preceding claims, wherein a molded part (G1, G2, G3) is incorporated in the algae and/or microorganism culture container.

6. The module (D) according to claim 5, wherein the molded part (G1, G2, G3) comprises pipes making it possible to supply the algae and/or microorganism culture container.

7. Module (D) according to any of the preceding claims, wherein the algae (W) are selected from the group comprising Chlorophyceae, Chlorella, Parietochloris incisa, Diatoms Amphora sp., Nitzchia sp., Chaetoceros sp.

8. Module (D) according to any of the preceding claims, wherein each wall independently comprises a single (M₁), double (M₂) or triple (M₃) glazing, a multi-wall glazing, or an assembly of these different glazing, the said multi-layer walls defining one or more air space.

9. Use of a photobioreactor (PBR) module (D) for insulation and/or thermal regulation according to any of claims 1 to 8 for the manufacture of a building.

10. A building facade comprising a photobioreactor (PBR) module (D) for insulation and/or thermal control according to any of claims 1 to 8.

11. A building comprising a plurality of photobioreactor (PBR) modules (D) for insulation and/or thermal control as defined in any one of claims 1 to 8.
